# EUROPEAN PATENT SPECIFICATION

(11) **EP 3 452 580 B1**
(45) Date of publication and mention of the grant of the patent: **16.08.2023**
(21) Application number: 17793067.4
(22) Date of filing: 01.05.2017
(51) Int. Cl.: C12N 5/0783, C12N 5/10, A61K 35/17, C07K 16/28, C07K 14/55, A61P 35/00, A61K 39/00

(54) **COMPOSITIONS AND METHODS FOR IMPROVED NK CELL THERAPIES**
ZUSAMMENSETZUNGEN UND VERFAHREN FÜR VERBESSERTE NK-ZELLEN-THERAPIEN
COMPOSITIONS ET MÉTHODES POUR THÉRAPIES PAR CELLULES NK AMÉLIORÉES

(30) Priority: 02.05.2016 US 201662330819 P
(43) Date of publication of application: 13.03.2019
(73) Proprietor: Cerus Corporation, Concord, CA 94520 (US)
(72) Inventor: STASSINOPOULOS, Adonis, Dublin CA 94568 (US); GREENMAN, William, Lafayette CA 94549 (US)
(74) Representative: Schiweck Weinzierl Koch Patentanwälte Partnerschaft mbB
(86) International application number: PCT/US2017/030385
(87) International publication number: WO 2017/192440

(56) References cited:
- WO-A1-2015/193411
- WO-A1-2016/073381
- US-A- 5 462 733
- SCHÖNFELD K ET AL: "Selective Inhibition of Tumor Growth by Clonal NK Cells Expressing an ErbB2/HER2-Specific Chimeric Antigen Receptor", MOLECULAR THERAPY, vol. 23, no. 2, 6 November 2014 (2014-11-06), pages 330-338, XP055265087, ISSN: 1525-0016, DOI: 10.1038/mt.2014.219
- WOLNICKA-GLUBISZ A ET AL.: "Effect of UVA and 8-methoxypsoralen, 4, 6, 4'-trimethylangelicin or chlorpromazine on apoptosis of lymphocytes and their recognition by monocytes", JOURNAL OF PHYSIOLOGY AND PHARMACOLOGY., vol. 61, no. 1, February 2010 (2010-02), pages 107-114, XP055650532, ISSN: 0867-5910
- BECHERUCCI V ET AL: "Extracorporeal photopheresis as an immunomodulatory agent: Haematocrit-dependent effects on natural killer cells : A New Suggested Immunomodulatory Effects of ECP", JOURNAL OF CLINICAL APHERESIS, vol. 32, no. 4, August 2016 (2016-08), pages 257-265, XP055650510, ISSN: 0733-2459, DOI: 10.1002/jca.21490
- KRAEMER et al.: "Effects of psoralens plus ultraviolet radiation on human lymphoid cells in vitro", National Cancer Institute monograph, vol. 66, December 1984 (1984-12), pages 221-223, XP009513810, Bethesda, Md. ISSN: 0083-1921
- BAKKOUR S ET AL: "Assessment of nucleic acid modification induced by amotosalen and ultraviolet A light treatment of platelets and plasma using real-time polymerase chain reaction amplification of variable length fragments of mitochondrial DNA.", TRANSFUSION, vol. 56, no. 2, February 2016 (2016-02), pages 410-420, ISSN: 1537-2995
- MARIAL SOBRAL P ET AL: "Viral inactivation in hemotherapy: systematic review on inactivators with action on nucleic acids.", REVISTA BRASILEIRA DE HEMATOLOGIA E HEMOTERAPIA, vol. 34, no. 3, 2012, pages 231-235, ISSN: 1806-0870
- HENSCHLER R ET AL: "Development of the S-303 Pathogen Inactivation Technology for Red Blood Cell Concentrates.", TRANSFUSION MEDICINE AND HEMOTHERAPY, vol. 38, no. 1, 2011, pages 33-42, ISSN: 1660-3796
- DREW V J ET AL: "Towards pathogen inactivation of red blood cells and whole blood targeting viral DNA/RNA: design, technologies, and future prospects for developing countries.", BLOOD TRANSFUSION, vol. 15, no. 6, October 2017 (2017-10), pages 512-521, ISSN: 2385-2070

## Description

### TECHNICAL FIELD

The present disclsoure relates to the preparation and use in recipients of CAR-NK cells which are modified by a nucleic acid targeting compound.

### BACKGROUND

Cell based immune therapies are becoming increasingly important for the treatment of diseases, including cancers. While donor T cell-based therapies may be more advanced, natural killer (NK) cell-based therapies are an emerging approach for immunotherapy. Recently, NK cells have been genetically engineered to produce chimeric antigen receptors, or CARs, on their surface (Schnofeld et al., 2015, Mol. Ther. 23:330-338; Hermanson et al., 2015, Front. Immunol. 6:195). CARs are proteins that can redirect NK cells and allow the cells to recognize a specific, pre-selected protein, or antigen, found on targeted cells, such as for example, tumor cells. CAR-NK cells can be cultured and expanded in the laboratory, then infused (*e.g*., re-infused) to patients. Through the guidance of the engineered cell receptor, CAR-NK cells recognize and destroy the cells (*e.g.*, cancer cells) that display the specific antigen on their surfaces.

Safety consideration for NK cell therapies can include, for example, on-target/off tumor effects, graft-versus-host-disease (GVHD) (*e.g*., including from cells and T cells or feeder cells), cytokine release leading to toxicity, tumor lysis syndrome, and toxicity to nomrmal tissues (Rezvani et al., 2015, Front. Immunol. 6:578). There remains a need in the art to provide improved methods and compositions for CAR-expressing NK cell therapies, particularly for the safety of these novel medical interventions.

### SUMMARY

The present disclosure relates to the preparation and use in recipients of CAR natural killer (NK) cell-derived effector cells which are modified by reaction of the nucleic acid of the NK cells with a nucleic acid targeting compound that reacts directly with the nucleic acid, for example, to limit proliferation of the cells within the recipient. This is accomplished through the introduction of crosslinks (*e.g*., interstrand crosslinks) and/or adducts into the genomic nucleic acids of CAR-NK cell-derived effector cells following expansion *in vitro* which prevent further division of the expanded CAR-NK cell-derived effector cells (*e.g*., activated CAR-NK cell-derived effector cells). Because some degree of function (*e.g*., cytokine release, cytotoxic activity) is likely a necessary consequence of NK cell activation and therefore efficacy, of CAR-NK cell-based therapy, the adducts are introduced with a frequency necessary to prevent cell division (and so further NK cell proliferation), but that permits the CAR-NK cell-derived effector cells to retain certain functions, such as for example immunologic function (*e.g*., complete immunologic function, partial immunologic function), including in certain embodiments cytoxic activity and/or the expression of one or more effector cytokines.

In a first aspect, the present disclosure provides a CAR-NK cell-derived effector cell population, comprising a population of NK cells (*e.g*., activated NK cells), such as for example, autologous NK cells, allogeneic NK cells, NK cell line, expressing a chimeric antigen receptor (CAR), the CAR comprising an extracellular domain which specifically binds a predetermined antigen (*e.g*., targeted antigen). In certain embodiments, the CAR comprises an extracellular domain which specifically binds a predetermined targeted antigen, a transmembrane domain, and a cytoplasmic co-stimulatory signaling domain. The nucleic acids of the NK cells have been modified by reaction with a nucleic acid targeting compound that reacts directly with the nucleic acid. In certain embodiments, the population of NK cells expressing a chimeric antigen receptor comprises a population of activated NK cells expressing a chimeric antigen receptor. In certain embodiments, the reaction of the nucleic acid of the NK cells with the nucleic acid targeting compound results in crosslinks (*e.g*., interstrand crosslinks) and/or adducts in the nucleic acid. In certain embodiments, the nucleic acid of the NK cells have been modified by reaction with the nucleic acid targeting compound so that the NK cells are attenuated for proliferation.

In various embodiments, the NK cells (*e.g*., activated NK cells) are present in the population in a therapeutically effective amount for treatment of disease or condition (*e.g*., malignancy, viral infection) that is associated with expression of the predetermined antigen. In certain embodiments, the NK cells are present in the population in a therapeutically effective amount for treatment of a malignancy that expresses the predetermined antigen. In certain embodiments, the NK cells are present in the population in a therapeutically effective amount for treatment of a viral infection that expresses the predetermined antigen. In preferred embodiments, the NK cells are activated NK cells and are provided in a pharmaceutically acceptable excipient which supports maintenance of the activated NK cells. Suitable buffers and salts are well known in the art for maintenance and administration of NK cells for adoptive cell transfer.

The term "attenuated for proliferation" as used herein refers to proliferation being inhibited in at least 50% of the CAR-NK cell-derived effector cells. In certain embodiments, the nucleic acid targeting agent is present in an amount effective to form from about 10² to about 10⁴ adducts per 10⁸ base pairs of genomic DNA of the NK cells. Preferably, the method results in proliferation being inhibited in at least 75%, at least 90%, at least 95%, at least 99%, at least 99.9%, or at least 99.99% or more of the CAR-NK cell-derived effector cells. Suitable nucleic acid targeting compounds or agents comprise, for example, an alkylator selected from the group consisting of mustards, mustard intermediates and mustard equivalents; a nucleic acid targeting group selected from the group consisting of intercalators, minor groove binders, major groove binders, electrostatic binders, and sequence-specific binders; FRALES (frangible anchor linker effector; see *e.g*., U.S. Patent 6,093,725) such as β-alanine, N-(acridin-9-yl), 2-[bis(2-chloroethyl)amino]ethyl ester; or a photoactivatable moiety selected from the group consisting of furocoumarins, actinomycins, anthracyclinones, anthramycins, benzodipyrones, fluorenes, fluorenones, monostral fats blue, norphillin A, organic dyes; phenanthridines, phenazathionium salts, phenazines, phenothiazines, phenylazides, quinolines and thiaxanthenones acridines and ellipticenes. In some embodiments, the nucleic acid targeting compound compound is a photoactive compound selected from the group consisting of a psoralen, an isoalloxazine, an alloxazine, a phthalocyanine, a phenothiazine, a porphyrin, and merocyanine 540. Other photoactive compounds known in the art include, for example, those set forth in U.S. Patent 5,593,823. In some preferred embodiments, the nucleic acid targeting compounds are psoralen compounds activated by UVA irradiation. In a preferred embodiment, the psoralen is 4'-(4-amino-2-oxa)butyl-4,5',8-trimethylpsoralen, 4'-aminomethyl 4, 5', 8-trimethylpsoralen (AMT), 5-methoxy psoralen, trioxalen, 4,5',8-trimethylpsoralen, or 8-methoxy psoralen. In some embodiments, the nucleic acid targeting compound is amotosalen (*e.g*., 3-(2-aminoethoxymethyl)-2,5,9-trimethylfuro[3,2-g]chromen-7-one and any salts thereof). This list is not meant to be limiting.

Most preferably, the nucleic acid targeting compound is activated by illumination (*e.g.*, irradiation), which may be a psoralen compound activated by UVA illumination. In some embodiments, the CAR-NK cell-derived effector cell population can comprise psoralen-induced interstrand crosslinks introduced between the strands of the genomic DNA double helix (*e.g*., interstrand crosslinks that inhibit replication of the NK cells as described hereinafter). In some embodiments, the CAR-NK cell-derived effector cell population can comprise FRALE-induced adducts introduced into the genomic DNA (*e.g*., adducts that inhibit replication of the NK cells as described hereinafter).

In some embodiments, at least a portion of the CAR-NK cells (*e.g*., activated CAR-NK cells) of the present disclosure may produce one or more cytokines, such as one or more cytokines selected from the group consisting of IFN-γ, GM-CSF, TNF and IL-10. Additionally, at least a portion of the CAR-NK cells (*e.g*., activated CAR-NK cells) may express one or more surface markers selected from the group consisting of CD56 (*e.g*., CD56^{bright}, CD56^{dim}), CD16, CD27 and NKp46.

When an antitumor chimeric antigen receptor is utilized, the tumor may be of any kind as long as it has a cell surface antigen which may be recognized by the chimeric receptor. In certain embodiments, a CAR-NK cell-derived effector cell population targets a cancer selected from the group consisting of lung cancer, melanoma, breast cancer, pancreatic cancer, gastric cancer, hepatocellular carcinoma, prostate cancer, colon cancer, renal cell carcinoma, ovarian cancer, neuroblastoma, rhabdomyosarcoma, leukemia (*e.g*., childhood acute lymphoblastic leukemia) and lymphoma (*e.g*., Hodgkin's lymphoma). In various embodiments, the predetermined antigen is a cancer antigen, and preferably is selected from the antigens listed in Table 1.

When an antiviral chimeric antigen receptor is utilized, the viral target may be of any kind as long as a viral antigen of the viral target is expressed on the surface of the cell (*e.g*., infected cell) which may be recognized by the chimeric receptor. In certain embodiments, a CAR-NK cell-derived effector cell population targets a viral agent selected from HIV or EBV. In certain embodiments, a CAR-NK cell-derived effector cell population targets an immune cell (*e.g*., T cell, B cell) expressing a viral antigen on the cell surface.

In some embodiments, the CAR-NK cell-derived effector cell population may further comprise a population of antigen presenting cells expressing the predetermined antigen, or a portion thereof. In some embodiments, the CAR-NK cell-derived effector cell population may further comprise a CAR-T cell-derived effector cell population comprising a population of activated T cells expressing a CAR, the CAR comprising an extracellular domain which specifically binds a predetermined antigen. In some embodiments, the nucleic acid of the T cells have been modified by reaction with a nucleic acid targeting compound that reacts directly with the nucleic acid. In some embodiments, the predeterimed antigen specifically bound by the the CAR of the CAR-T cell-derived effector cell population is the same as the predeterimed antigen specifically bound by the the CAR of the CAR-NK cell-derived effector cell population. In some embodiments, the predeterimed antigen specifically bound by the the CAR of the CAR-T cell-derived effector cell population is different from the predeterimed antigen specifically bound by the the CAR of the CAR-NK cell-derived effector cell population.

In a related aspect, the present disclosure provides a method of inducing an immune response to at least one predetermined antigen in a subject, comprising administering to the subject a CAR-NK cell-derived effector cell population as described herein in an amount sufficient to induce an anti-tumor response to a cancer in the subject, wherein the cancer expresses the predetermined antigen. In some embodiments, the cancer is selected from the group consisting of lung cancer, melanoma, breast cancer, pancreatic cancer, gastric cancer, hepatocellular carcinoma, prostate cancer, colon cancer, renal cell carcinoma, ovarian cancer, neuroblastoma, rhabdomyosarcoma, leukemia (*e.g*., childhood acute lymphoblastic leukemia) and lymphoma (*e.g*., Hodgkin's lymphoma). In various embodiments, the predetermined antigen is a cancer antigen, and preferably is selected from the antigens listed in Table 1. In some embodiments, the immune response is a cytotoxic anti-tumor response. In some embodiments, the method further comprises inducing tumor cell apoptosis. In some embodiments, the method further comprises administering to the subject a CAR-T cell-derived effector cell population comprising a population of activated T cells expressing a CAR, the CAR comprising an extracellular domain which specifically binds a predetermined antigen, wherein the nucleic acid of the T cells have been modified by reaction with a nucleic acid targeting compound that reacts directly with the nucleic acid. In some embodiments, the predeterimed antigen specifically bound by the the CAR of the CAR-T cell-derived effector cell population is the same as the predeterimed antigen specifically bound by the the CAR of the CAR-NK cell-derived effector cell population. In some embodiments, the predeterimed antigen specifically bound by the the CAR of the CAR-T cell-derived effector cell population is different from the predeterimed antigen specifically bound by the the CAR of the CAR-NK cell-derived effector cell population.

In another related aspect, the present disclosure provides a method to enhance an anti-tumor immune response (*e.g*., T cell response) to a cancer in the subject, comprising administering to the subject a CAR-NK cell-derived effector cell population as described herein in an amount sufficient to enhance the anti-tumor response to the cancer. In some embodiments, the CAR of the CAR-NK cell-derived effector cell population comprises an extracellular domain that specifically binds to a predetermined antigen, wherein the cancer expresses the predetermined antigen. In some embodiments, the anti-tumor immune response is a T cell response induced by a T cell population present in the subject or present in the CAR-NK cell-derived effector cell population. In some embodiments, the anti-tumor immune response is a T cell response induced by administering to the same subject a CAR-T cell-derived effector cell population comprising a population of activated T cells expressing a CAR, the CAR comprising an extracellular domain which specifically binds the predetermined antigen. In some embodiments, the anti-tumor immune response is a T cell response induced by administering to the same subject a CAR-T cell-derived effector cell population comprising a population of activated T cells expressing a CAR, the CAR comprising an extracellular domain which specifically binds to a predetermined antigen that is different than the CAR-NK cell-derived effector cell population.

In another aspect, the present disclosure provides a method of preparing a CAR-NK cell-derived cell population comprising a population of NK cells expressing a chimeric antigen receptor (CAR), the CAR comprising an extracellular domain which specifically binds a predetermined antigen, comprising:
contacting in vitro one or more NK cells that have been modified to express the CAR with a stimulus that induces expansion of the NK cells to provide an expanded NK cell population; and
modifying the nucleic acid of the NK cells in the expanded NK cell population by reaction with a nucleic acid targeting compound that reacts directly with the nucleic acid.

In some embodiments, the NK cells in the expanded NK cell population are attenuated for proliferation. In some embodiments, the method further comprises, prior to or following the modifying step, activating in vitro the NK cells to produce a CAR-NK cell-derived effector cell population. In some embodiments, the NK cells in the effector NK cell population are attenuated for proliferation.

As described herein, the CAR-NK cells of the present disclosure may be expanded and activated *in vitro* to provide a sufficient CAR-NK cell-derived effector cell population that is attenuated for further proliferation *in vivo* in the subject receiving the CAR-NK cell therapy. The expansion step necessarily precedes modification of the nucleic acid which renders the cells attenuated for proliferation. The activation step, however, may precede or follow modification of the nucleic acid. Thus, in certain embodiments, the method comprises contacting the one or more NK cells with the predetermined antigen under conditions in which the NK cells are both induced to expand and are activated by the same stimulus, and the NK cells in the expanded NK cell population are attenuated for proliferation following expansion and activation. In alternative embodiments, the stimulus that induces expansion of the NK cells is a non-specific expansion stimulus, and the expanded NK cell population is subsequently activated by contacting the NK cells in the expanded NK cell population with the predetermined antigen under conditions in which the NK cells are activated. In these latter embodiments, the NK cells in the expanded NK cell population may be attenuated for proliferation either before or following the activation step. In alternative embodiments, the stimulus that induces expansion and activation of the NK cells is a non-specific expansion stimulus. In some embodiments, the NK cells in the expanded NK cell population are attenuated for proliferation following expansion and activation. In some embodiments, the stimulus comprises one or more of IL-2, IL-12, IL-15 IL-18 and IL-21.

In some embodiments, the nucleic acid targeting compound is a nucleic acid alkylator. In some embodiments, the nucleic acid alkylator is a FRALE such as β-alanine, N-(acridin-9-yl), 2-[bis(2-chloroethyl)amino] ethyl ester. In some embodiments, the nucleic acid targeting compound is activated by illumination. In some embodiments, the nucleic acid targeting compound is a psoralen compound activated by UVA illumination. In some embodiments, the NK cells comprise psoralen-induced interstrand crosslinks introduced between the strands of the genomic DNA. In some embodiments, the crosslinks inhibit replication of the NK cells. In some embodiments, the psoralen is 4'-(4-amino-2-oxa)butyl-4,5',8-trimethylpsoralen, 4'aminomethyl 4, 5', 8trimethylpsoralen (AMT), 5-methoxy psoralen, trioxalen 4, 5' 8-trimethylpsoralen, or 8-methoxy psoralen. In some embodiments, at least a portion of the NK cells produce one or more cytokines. In some embodiments, at least a portion of the NK cells produce one or more cytokines selected from the group consisting of IFN-γ, GM-CSF, TNF and IL-10. In some embodiments, at least a portion of the NK cells express one or more surface markers selected from the group consisting of CD56 (e.g., CD56bright, CD56dim), CD16, CD27 and NKp46. In some embodiments, greater than 90% of the NK cells in the population are non-proliferating. In some embodiments, greater than 90% of the activated NK cells in the population are non-proliferating. In some embodiments, the predetermined antigen is a cancer antigen. In some embodiments, the predetermined antigen is selected from the antigens listed in Table 1. In some embodiments, the cancer is selected from the group consisting of lung cancer, melanoma, breast cancer, prostate cancer, colon cancer, renal cell carcinoma, ovarian cancer, neuroblastoma, rhabdomyosarcoma, leukemia and lymphoma.

It is to be understood that the disclosure is not limited in its application to the details of construction and to the arrangements of the components set forth in the following description or illustrated in the drawings. The disclosure provided is capable of embodiments in addition to those described and of being practiced and carried out in various ways. Also, it is to be understood that the phraseology and terminology employed herein, as well as the abstract, are for the purpose of description and should not be regarded as limiting.

### DETAILED DESCRIPTION

The present disclosure relates to the preparation and use of CAR-NK cells, including for example, CAR-NK cells which exhibit a reduced propensity for expansion (*e.g*., uncontrolled expansion) *in vivo* following administration to a subject. The CAR-NK cells may also offer other safety advantages as provided herein. As set forth in some embodiments, by inducing activation and proliferation *ex vivo* and treating the cells so as to inhibit further proliferation *in vivo* following administration, the CAR-NK cell-derived effector cells of the present disclosure retain the ability to specifically target disease, but with reduced complications.

"CAR-NK cells" refer to a NK cell or population thereof, which has been modified through molecular biological methods to express a chimeric antigen receptor (CAR) on the NK cell surface. The CAR is a polypeptide having a pre-defined binding specificity to a desired target expressed operably connected to (*e.g*., as a fusion, separate chains linked by one or more disulfide bonds, etc.) the intracellular part of a T-cell activation domain. By bypassing MHC class I and class II restriction, CAR engineered NK cells can be recruited for redirected target cell recognition. The most common CARs are fusions of immuoglobulin binding functionality (e.g., as a single-chain variable fragment (scFv) derived from a monoclonal antibody) to CD3-zeta (CD3ζ) transmembrane and endodomain. Such molecules result in the transmission of a zeta signal in response to recognition by the immuoglobulin binding functionality of its target. There are, however, many alternatives. By way of example, an antigen recognition domain from native T-cell receptor (TCR) alpha and beta single chains may be used as the binding functionality. Alternatively, receptor ectodomains (*e.g*. CD4 ectodomain) or cytokines (which leads to recognition of cells bearing the cognate cytokine receptor) may be employed. All that is required of the binding functionality is that it binds a given target with high affinity in a specific manner.

"Specifically" or "selectively" binds, when referring to a ligand/receptor, nucleic acid/complementary nucleic acid, antibody/antigen, or other binding pair (*e.g.*, a cytokine to a cytokine receptor) indicates a binding reaction which is determinative of the presence of the protein in a heterogeneous population of proteins and other biologics. Thus, under designated conditions, a specified ligand binds to a particular receptor and does not bind in a significant amount to other proteins present in the sample. Specific binding can also mean, e.g., that the binding compound, nucleic acid ligand, antibody, or binding composition derived from the antigen-binding site of an antibody, of the contemplated method binds to its target with an affinity that is often at least 25% greater, more often at least 50% greater, most often at least 100% (2-fold) greater, normally at least ten times greater, more normally at least 20-times greater, and most normally at least 100-times greater than the affinity with any other binding compound.

In a typical embodiment a molecule that specifically binds a target will have an affinity that is at least about 10⁶ liters/mol (*K*_{D} = 10⁻⁶ M), and preferably at least about 10⁸ liters/mol, as determined, e.g., by Scatchard analysis (Munsen, et al. (1980) Analyt. Biochem. 107:220-239). It is recognized by the skilled artisan that some binding compounds can specifically bind to more than one target, e.g., an antibody specifically binds to its antigen, to lectins by way of the antibody's oligosaccharide, and/or to an Fc receptor by way of the antibody's Fc region.

It is to be understood that the disclosure is not limited in its application to the details of construction and to the arrangements of the components set forth in the following description or illustrated in the drawings. The disclosure provided herein is capable of embodiments in addition to those described and of being practiced and carried out in various ways. Also, it is to be understood that the phraseology and terminology employed herein, as well as the abstract, are for the purpose of description and should not be regarded as limiting.

### 1. Definitions

"Administration" as it applies to a human, primate, mammal, mammalian subject, animal, veterinary subject, placebo subject, research subject, experimental subject, cell, tissue, organ, or biological fluid, refers without limitation to contact of an exogenous ligand, reagent, placebo, small molecule, pharmaceutical agent, therapeutic agent, diagnostic agent, or composition to the subject, cell, tissue, organ, or biological fluid, and the like. "Administration" can refer, *e.g.*, to therapeutic, pharmacokinetic, diagnostic, research, placebo, and experimental methods. Treatment of a cell encompasses contact of a reagent to the cell, as well as contact of a reagent to a fluid, where the fluid is in contact with the cell. "Administration" also encompasses *in vitro* and *ex vivo* treatments, *e.g.*, of a cell, by a reagent, diagnostic, binding composition, or by another cell.

An "agonist," as it relates to a ligand and receptor, comprises a molecule, combination of molecules, a complex, or a combination of reagents, that stimulates the receptor. For example, an agonist of granulocyte-macrophage colony stimulating factor (GM-CSF) can encompass GM-CSF, a mutein or derivative of GM-CSF, a peptide mimetic of GM-CSF, a small molecule that mimics the biological function of GM-CSF, or an antibody that stimulates GM-CSF receptor.

An "antagonist," as it relates to a ligand and receptor, comprises a molecule, combination of molecules, or a complex, that inhibits, counteracts, downregulates, and/or desensitizes the receptor. "Antagonist" encompasses any reagent that inhibits a constitutive activity of the receptor. A constitutive activity is one that is manifest in the absence of a ligand/receptor interaction. "Antagonist" also encompasses any reagent that inhibits or prevents a stimulated (or regulated) activity of a receptor. By way of example, an antagonist of GM-CSF receptor includes, without implying any limitation, an antibody that binds to the ligand (GM-CSF) and prevents it from binding to the receptor, or an antibody that binds to the receptor and prevents the ligand from binding to the receptor, or where the antibody locks the receptor in an inactive conformation.

As used herein, an "analog" or "derivative" with reference to a peptide, polypeptide or protein refers to another peptide, polypeptide or protein that possesses a similar or identical function as the original peptide, polypeptide or protein, but does not necessarily comprise a similar or identical amino acid sequence or structure of the original peptide, polypeptide or protein. An analog preferably satisfies at least one of the following: (a) a proteinaceous agent having an amino acid sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the original amino acid sequence (b) a proteinaceous agent encoded by a nucleotide sequence that hybridizes under stringent conditions to a nucleotide sequence encoding the original amino acid sequence; and (c) a proteinaceous agent encoded by a nucleotide sequence that is at least 30%, at least 35%, at least 40%, at least 45%, at least 50%, at least 55%, at least 60%, at least 65%, at least 70%, at least 75%, at least 80%, at least 85%, at least 90%, at least 95% or at least 99% identical to the nucleotide sequence encoding the original amino acid sequence.

"Antigen presenting cells" (APCs) are cells of the immune system used for presenting antigen to other cells (*e.g*., T cells, NK cells). APCs include, for example, dendritic cells, monocytes, macrophages, marginal zone Kupffer cells, microglia, Langerhans cells, T cells, and B cells. Dendritic cells occur in at least two lineages. The first lineage encompasses pre-DC1, myeloid DC1, and mature DC1. The second lineage encompasses CD34⁺CD45RA⁻ early progenitor multipotent cells, CD34⁺CD45RA⁺ cells, CD34⁺CD45RA⁺CD4⁺ IL-3Rα⁺ pro-DC2 cells, CD4⁺CD11c⁻ plasmacytoid pre-DC2 cells, lymphoid human DC2 plasmacytoid-derived DC2s, and mature DC2s.

"Attenuation" and "attenuated" encompasses a bacterium, virus, parasite, infectious organism, prion, cell (*e.g*., tumor cell, NK cell, T cell), gene in an infectious organism, and the like, that is modified to reduce toxicity to a host or its ability to proliferate with potential undesirable effects. The host can be a human or animal host, or an organ, tissue, or cell. The bacterium, to give a non-limiting example, can be attenuated to reduce binding to a host cell, to reduce spread from one host cell to another host cell, to reduce extracellular growth, or to reduce intracellular growth in a host cell. Attenuation can be assessed by measuring, e.g., an indicum or indicia of toxicity, the LD₅₀, the rate of clearance from an organ, or the competitive index (see, e.g., Auerbuch, et al. (2001) Infect. Immunity 69:5953-5957). Generally, an attenuation results an increase in the LD₅₀ and/or an increase in the rate of clearance by at least 25%; more generally by at least 50%; most generally by at least 100% (2-fold); normally by at least 5-fold; more normally by at least 10-fold; most normally by at least 50-fold; often by at least 100-fold; more often by at least 500-fold; and most often by at least 1000-fold; usually by at least 5000-fold; more usually by at least 10,000-fold; and most usually by at least 50,000-fold; and most often by at least 100,000-fold. Generally, attenuation of cell (*e.g.*, NK cell, T cell) proliferation results in a decrease in proliferation by at least 50%, at least 75%, at least 90%, at least 95%, at least 98%, at least 99%, at least 99.9%, at least 99.99% or more, or at least 10-fold, at least 100-fold, at least 1000-fold, at least 10,000-fold, or at least 100,000-fold or more.

"Effective amount" encompasses, without limitation, an amount that can ameliorate, reverse, mitigate, prevent, or diagnose a symptom or sign of a medical condition or disorder. Unless dictated otherwise, explicitly or by context, an "effective amount" is not limited to a minimal amount sufficient to ameliorate a condition.

An "extracellular fluid" encompasses, e.g., serum, plasma, blood, interstitial fluid, cerebrospinal fluid, secreted fluids, lymph, bile, sweat, fecal matter, and urine. An "extracelluar fluid" can comprise a colloid or a suspension, e.g., whole blood or coagulated blood.

The term "fragments" in the context of polypeptides include a peptide or polypeptide comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, at least 150 contiguous amino acid residues, at least 175 contiguous amino acid residues, at least 200 contiguous amino acid residues, or at least 250 contiguous amino acid residues of the amino acid sequence of a larger polypeptide.

"Gene" refers to a nucleic acid sequence encoding an oligopeptide or polypeptide. The oligopeptide or polypeptide can be biologically active, antigenically active, biologically inactive, or antigenically inactive, and the like. The term gene encompasses, e.g., the sum of the open reading frames (ORFs) encoding a specific oligopeptide or polypeptide; the sum of the ORFs plus the nucleic acids encoding introns; the sum of the ORFs and the operably linked promoter(s); the sum of the ORFS and the operably linked promoter(s) and any introns; the sum of the ORFS and the operably linked promoter(s), intron(s), and promoter(s), and other regulatory elements, such as enhancer(s). In certain embodiments, "gene" encompasses any sequences required in cis for regulating expression of the gene. The term gene can also refer to a nucleic acid that encodes a peptide encompassing an antigen or an antigenically active fragment of a peptide, oligopeptide, polypeptide, or protein. The term gene does not necessarily imply that the encoded peptide or protein has any biological activity, or even that the peptide or protein is antigenically active. A nucleic acid sequence encoding a non-expressable sequence is generally considered a pseudogene. The term gene also encompasses nucleic acid sequences encoding a ribonucleic acid such as rRNA, tRNA, or a ribozyme.

A composition that is "labeled" is detectable, either directly or indirectly, by spectroscopic, photochemical, biochemical, immunochemical, isotopic, or chemical methods. For example, useful labels include ³²P, ³³P, ³⁵S, ¹⁴C, ³H, ¹²⁵I, stable isotopes, epitope tags, fluorescent dyes, electron-dense reagents, substrates, or enzymes, e.g., as used in enzyme-linked immunoassays, or fluorettes (see, e.g., Rozinov and Nolan (1998) Chem. Biol. 5:713-728).

"Ligand" refers to a small molecule, peptide, polypeptide, or membrane associated or membrane-bound molecule that is an agonist or antagonist of a receptor. "Ligand" also encompasses a binding agent that is not an agonist or antagonist, and has no agonist or antagonist properties. By convention, where a ligand is membrane-bound on a first cell, the receptor usually occurs on a second cell. The second cell may have the same identity (the same name), or it may have a different identity (a different name), as the first cell. A ligand or receptor may be entirely intracellular, that is, it may reside in the cytosol, nucleus, or in some other intracellular compartment. The ligand or receptor may change its location, e.g., from an intracellular compartment to the outer face of the plasma membrane. The complex of a ligand and receptor is termed a "ligand receptor complex." Where a ligand and receptor are involved in a signaling pathway, the ligand occurs at an upstream position and the receptor occurs at a downstream position of the signaling pathway.

"Nucleic acid" refers to deoxyribonucleotides or ribonucleotides and polymers thereof in either single stranded, double-stranded form, or multi-stranded form. Non-limiting examples of a nucleic acid are a, *e.g.*, cDNA, mRNA, oligonucleotide, and polynucleotide. A particular nucleic acid sequence can also implicitly encompass "allelic variants" and "splice variants."

"Operably linked" in the context of a promoter and a nucleic acid encoding a mRNA means that the promoter can be used to initiate transcription of that nucleic acid.

The terms "percent sequence identity" and "% sequence identity" refer to the percentage of sequence similarity found by a comparison or alignment of two or more amino acid or nucleic acid sequences. Percent identity can be determined by a direct comparison of the sequence information between two molecules by aligning the sequences, counting the exact number of matches between the two aligned sequences, dividing by the length of the shorter sequence, and multiplying the result by 100. An algorithm for calculating percent identity is the Smith-Waterman homology search algorithm (see, e.g., Kann and Goldstein (2002) Proteins 48:367-376; Arslan, et al. (2001) Bioinformatics 17:327-337).

By "purified" and "isolated" is meant, when referring to a polypeptide, that the polypeptide is present in the substantial absence of the other biological macromolecules with which it is associated in nature. The term "purified" as used herein means that an identified polypeptide often accounts for at least 50%, more often accounts for at least 60%, typically accounts for at least 70%, more typically accounts for at least 75%, most typically accounts for at least 80%, usually accounts for at least 85%, more usually accounts for at least 90%, most usually accounts for at least 95%, and conventionally accounts for at least 98% by weight, or greater, of the polypeptides present. The weights of water, buffers, salts, detergents, reductants, protease inhibitors, stabilizers (including an added protein such as albumin), and excipients, and molecules having a molecular weight of less than 1000, are generally not used in the determination of polypeptide purity. See, e.g., discussion of purity in U.S. Pat. No. 6,090,611 issued to Covacci, et al.

"Peptide" refers to a short sequence of amino acids, where the amino acids are connected to each other by peptide bonds. A peptide may occur free or bound to another moiety, such as a macromolecule, lipid, oligo- or polysaccharide, and/or a polypeptide. Where a peptide is incorporated into a polypeptide chain, the term "peptide" may still be used to refer specifically to the short sequence of amino acids. A "peptide" may be connected to another moiety by way of a peptide bond or some other type of linkage. A peptide is at least two amino acids in length and generally less than about 25 amino acids in length, where the maximal length is a function of custom or context. The terms "peptide" and "oligopeptide" may be used interchangeably.

"Protein" generally refers to the sequence of amino acids comprising a polypeptide chain. Protein may also refer to a three dimensional structure of the polypeptide. "Denatured protein" refers to a partially denatured polypeptide, having some residual three dimensional structure or, alternatively, to an essentially random three dimensional structure, *i.e*., totally denatured. The disclosure encompasses reagents of, and methods using, polypeptide variants, e.g., involving glycosylation, phosphorylation, sulfation, disulfide bond formation, deamidation, isomerization, cleavage points in signal or leader sequence processing, covalent and non-covalently bound cofactors, oxidized variants, and the like. The formation of disulfide linked proteins is described (see, e.g., Woycechowsky and Raines (2000) Curr. Opin. Chem. Biol. 4:533-539; Creighton, et al. (1995) Trends Biotechnol. 13:18-23).

"Recombinant" when used with reference, *e.g.*, to a nucleic acid, cell, animal, virus, plasmid, vector, or the like, indicates modification by the introduction of an exogenous, non-native nucleic acid, alteration of a native nucleic acid, or by derivation in whole or in part from a recombinant nucleic acid, cell, virus, plasmid, or vector. Recombinant protein refers to a protein derived, e.g., from a recombinant nucleic acid, virus, plasmid, vector, or the like. "Recombinant bacterium" encompasses a bacterium where the genome is engineered by recombinant methods, e.g., by way of a mutation, deletion, insertion, and/or a rearrangement. "Recombinant bacterium" also encompasses a bacterium modified to include a recombinant extra-genomic nucleic acid, e.g., a plasmid or a second chromosome, or a bacterium where an existing extra-genomic nucleic acid is altered.

"Sample" refers to a sample from a human, animal, placebo, or research sample, e.g., a cell, tissue, organ, fluid, gas, aerosol, slurry, colloid, or coagulated material. The "sample" may be tested *in vivo*, e.g., without removal from the human or animal, or it may be tested *in vitro.* The sample may be tested after processing, e.g., by histological methods. "Sample" also refers, e.g., to a cell comprising a fluid or tissue sample or a cell separated from a fluid or tissue sample. "Sample" may also refer to a cell, tissue, organ, or fluid that is freshly taken from a human or animal, or to a cell, tissue, organ, or fluid that is processed or stored.

A "selectable marker" encompasses a nucleic acid that allows one to select for or against a cell that contains the selectable marker. Examples of selectable markers include, without limitation, *e.g*.: (1) A nucleic acid encoding a product providing resistance to an otherwise toxic compound (*e.g*., an antibiotic), or encoding susceptibility to an otherwise harmless compound (e.g., sucrose); (2) A nucleic acid encoding a product that is otherwise lacking in the recipient cell (*e.g.*, tRNA genes, auxotrophic markers); (3) A nucleic acid encoding a product that suppresses an activity of a gene product; (4) A nucleic acid that encodes a product that can be readily identified (e.g., phenotypic markers such as beta-galactosidase, green fluorescent protein (GFP), cell surface proteins, an epitope tag, a FLAG tag); (5) A nucleic acid that can be identified by hybridization techniques, for example, PCR or molecular beacons.

The term "subject" as used herein refers to a human or non-human organism. Thus, the methods and compositions described herein are applicable to both human and veterinary disease. In certain embodiments, subjects are "patients," *i.e.*, living humans that are receiving medical care for a disease or condition. This includes persons with no defined illness who are being investigated for signs of pathology.

"Therapeutically effective amount" is defined as an amount of a reagent or pharmaceutical composition that is sufficient to induce a desired immune response (*e.g*., specific for encoded heterologous antigens), show a patient benefit, *i.e.*, to cause a decrease, prevention, or amelioration of the symptoms of the condition being treated. When the agent or pharmaceutical composition comprises a diagnostic agent, a "diagnostically effective amount" is defined as an amount that is sufficient to produce a signal, image, or other diagnostic parameter. Effective amounts of the pharmaceutical formulation will vary according to factors such as the degree of susceptibility of the individual, the age, gender, and weight of the individual, and idiosyncratic responses of the individual (see, *e.g.*, U.S. Pat. No. 5,888,530 issued to Netti, et al.)*.*

"Treatment" or "treating" (with respect to a condition or a disease) is an approach for obtaining beneficial or desired results including and preferably clinical results. For purposes of this disclosure, beneficial or desired results with respect to a disease include, but are not limited to, one or more of the following: improving a condition associated with a disease, curing a disease, lessening severity of a disease, delaying progression of a disease, alleviating one or more symptoms associated with a disease, increasing the quality of life of one suffering from a disease, and/or prolonging survival. Likewise, for purposes of this disclosure, beneficial or desired results with respect to a condition include, but are not limited to, one or more of the following: improving a condition, curing a condition, lessening severity of a condition, delaying progression of a condition, alleviating one or more symptoms associated with a condition, increasing the quality of life of one suffering from a condition, and/or prolonging survival.

### 2. CAR-NK cells

The term "chimeric receptor" as used herein refers to a cell-surface receptor comprising an extracellular ligand binding domain, a transmembrane domain and a cytoplasmic signaling domain (*e.g*., co-stimulatory signaling domain) in a combination that is not naturally found together on a single protein. This particularly includes receptors wherein the extracellular domain and the cytoplasmic domain are not naturally found together on a single receptor protein. As described in U.S. Pat. Nos. 5,359,046, 5,686,281 and 6,103,521, the extracellular domain may be obtained from any of the wide variety of extracellular domains or secreted proteins associated with ligand binding and/or signal transduction. The extracellular domain may be part of a protein which is monomeric, homodimeric, heterodimeric, or associated with a larger number of proteins in a non-covalent complex. In particular, the extracellular domain may consist of an Ig heavy chain which may in turn be covalently associated with Ig light chain by virtue of the presence of CH1 and hinge regions, or may become covalently associated with other Ig heavy/light chain complexes by virtue of the presence of hinge, CH2 and CH3 domains. In the latter case, the heavy/light chain complex that becomes joined to the chimeric construct may constitute an antibody with a specificity distinct from the antibody specificity of the chimeric construct. Depending on the function of the antibody, the desired structure and the signal transduction, the entire chain may be used or a truncated chain may be used, where all or a part of the CH1, CH2, or CH3 domains may be removed or all or part of the hinge region may be removed.

As described herein, the extracellular domains of CARs are often derived from immunoglobulins. The term "antibody" as used herein refers to a peptide or polypeptide derived from, modeled after or substantially encoded by an immunoglobulin gene or immunoglobulin genes, or fragments thereof, capable of specifically binding an antigen or epitope. *See*, *e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHl domains; (ii) a F(ab')2 fragment, a bivalent fragment comprising two Fab fragments linked by a disulfide bridge at the hinge region; (iii) a Fd fragment consisting of the VH and CHl domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See*, *e.g*, Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See*, *e.g.*, U.S. Patent No. 6,057,098,

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present. The antibodies so identified may then be further analyzed for affinity and specificity in the CAR design selected.

When an antitumor chimeric antigen receptor is utilized, the tumor may be of any kind as long as it has a cell surface antigen which may be recognized by the chimeric receptor. In a specific embodiment, the chimeric receptor may be for any cancer for which a specific monoclonal antibody exists or is capable of being generated. In particular, cancers such as neuroblastoma, small cell lung cancer, melanoma, ovarian cancer, renal cell carcinoma, colon cancer, Hodgkin's lymphoma, and acute lymphoblastic leukemia (*e.g*., childhood acute lymphoblastic leukemia) have antigens which may be targeted by the chimeric receptors. The compositions and methods of this disclosure can be used in immunotherapy in the treatment of cancer, in particular the treatment of lung cancer, melanoma, breast cancer, prostate cancer, colon cancer, renal cell carcinoma, ovarian cancer, neuroblastoma, rhabdomyosarcoma, leukemia and lymphoma. The compositions and methods described in the present disclosure may be utilized in conjunction with other types of therapy for cancer, such as chemotherapy, surgery, radiation, gene therapy, and so forth, as described hereinafter.

When an antiviral chimeric antigen receptor is utilized, the viral target may be of any kind as long as a viral antigen of the viral target is expressed on the surface of a cell (*e.g*., infected cell) which may be recognized by the chimeric receptor. In a specific embodiment, the chimeric receptor may be for any viral target for which a specific monoclonal antibody exists or is capable of being generated. In particular, viral antigens from enveloped viruses are particularly suitable for targeting by the chimeric receptors. In certain embodiments, the present disclosure provides a CAR-NK cell-derived effector cell population that targets a viral agent selected from HIV (Ni et al., 2014, Stem Cells 32:1021-1031) or EBV (Tassev et al., 2012, Cancer Gene Ther. 19:84-100). In certain embodiments, the present disclosure provides a CAR-NK cell-derived effector cell population that targets an immune cell (*e.g.*, T cell, B cell) expressing a viral antigen on the cell surface.

The transmembrane domain may be contributed by the protein contributing the multispecific extracellular inducer clustering domain, the protein contributing the effector function signaling domain, the protein contributing the proliferation signaling portion, or by a totally different protein. For the most part it will be convenient to have the transmembrane domain naturally associated with one of the domains. In some cases it will be desirable to employ the transmembrane domain of the ζ, η or FcεR1γ chains which contain a cysteine residue capable of disulfide bonding, so that the resulting chimeric protein will be able to form disulfide linked dimers with itself, or with unmodified versions of the ζ, η or FcεR1γ chains or related proteins. In some instances, the transmembrane domain will be selected or modified by amino acid substitution to avoid binding of such domains to the transmembrane domains of the same or different surface membrane proteins to minimize interactions with other members of the receptor complex. In other cases it will be desirable to employ the transmembrane domain of ζ, η or FcεR1γ chains and -β, MB1 (Igα), B29 or CD3y, ζ, or ε, in order to retain physical association with other members of the receptor complex. Examples of suitable transmembrane regions for use with the disclosure include the constant (Fc) regions of immunoglobins, human CD8a, and artificial linkers that serve to move the targeting moiety away from the cell surface for improved access to and binding on target cells, however any transmembrane region sufficient to anchor the CAR in the membrane can be used. Persons of skill are aware of numerous transmembrane regions and the structural elements (such as lipophilic amino acid regions) that produce transmembrane domains in numerous membrane proteins and therefore can substitute any convenient sequence.

The cytoplasmic domain of the chimeric receptors of the disclosure can comprise a signaling domain (*e.g*., co-stimulatory signaling domain) by itself or combined with any other desired cytoplasmic domain(s) useful in the context of this chimeric receptor type, such as for example, a 4-1BB signaling domain, a CD3ζ signaling domain and/or a CD28 signaling domain. The 4-1BB, CD3ζ and CD28 signaling domains are well characterized, including for example, their use in chimeric receptors. In one embodiment, the cytoplasmic domain of the chimeric receptors can comprise the 4-1BB signaling domain by itself or combined with any other desired cytoplasmic domain(s) useful in the context of this chimeric receptor type. For example, in one embodiment, the extracellular domain comprises a single chain variable domain of a monoclonal antibody, the transmembrane domain comprises the hinge and transmembrane domain of CD8α, and the cytoplasmic domain comprises the signaling domain of CD3ζ and the signaling domain of 4-1BB. The CD8α hinge and transmembrane domain consists of 69 amino acids translated from the 207 nucleotides at positions 815-1021 of GenBank Accession No. NM_001768. The CD3ζ signaling domain of the preferred embodiment contains 112 amino acids translated from 339 nucleotides at positions 1022-1360 of GenBank Accession No. NM_000734.

Genetic modification for introduction of the CAR construct into NK cells can be accomplished by transducing (or otherwise delivering) a NK cell composition (*e.g.*, autologous NK cells, allogeneic NK cells, NK cell line) with a recombinant DNA or RNA construct, such as for example, a vector. A vector may be any agent capable of delivering or maintaining nucleic acid in a host cell, and includes viral vectors (*e.g*. retroviral vectors, lentiviral vectors, adenoviral vectors, or adeno-associated viral vectors), plasmids, naked nucleic acids, nucleic acids complexed with polypeptide or other molecules and nucleic acids immobilized onto solid phase particles. The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

Selection of promoter and other regulatory sequences for protein expression are well known to those of skill in the art. Cell specific promoters for expression in T-cells include, but are not limited to, human CD2, distal Lck, and proximal Lck. In other embodiments, non-tissue specific promoters such as non-tissue specific promoters including viral promoters such as cytomegalovirus (CMV) promoter, β-actin promoter phosphoglycerate kinase (PGK) promoter, ubiquitin promoter, and EF-1α promoter can be used. This list is not meant to be limiting. An expression construction preferably also includes sequences to allow for the replication of the expression construct. Transcription of the DNA encoding the polypeptides of the present disclosure by higher eukaryotes may be increased by inserting an enhancer sequence into the vector. Enhancers are cis-acting elements of DNA, usually about from 10 to 300 by that act on a promoter to increase its transcription. Examples including the SV40 enhancer on the late side of the replication origin by 100 to 270, a cytomegalovirus early promoter enhancer, the polyoma enhancer on the late side of the replication origin, and adenovirus enhancers. Preferably, a retroviral vector (either gamma-retroviral or lentiviral) is employed for the introduction of the CAR nucleic acid construct into the cell. For example, a polynucleotide encoding a co-stimulatory ligand protein (e.g., tumor necrosis factor (TNF) ligand, such as 4-1BBL, OX40L, CD70, LIGHT, and CD30L, or an Ig superfamily ligand, such as CD80 and CD86), or a receptor that binds an antigen, or a variant, or a fragment thereof, can be cloned into a retroviral vector and expression can be driven from its endogenous promoter, from the retroviral long terminal repeat, or from a promoter specific for a target cell type of interest. Non-viral vectors may be used as well.

### 3. Inhibition of NK cell proliferation in recipients

The antigen-specific CAR-NK cells can be expanded *in vitro* for use in adoptive cellular immunotherapy infusions to achieve desired activity against target cell types (*e.g*., anti-tumor reactivity in a tumor-bearing host; antiviral reactivity against a viral antigen expressing cell population in a host). The CAR-NK cells of the present disclosure may be isolated, and then expanded and activated *in vitro* using methods known in the art to reach therapeutically sufficient numbers prior to administration to a subject (see *e.g.*, Glienke et al. 2015, Front. Pharmacol. 21:1-7). The NK cells generally may be expanded non-specifically with one or more cytokines (*e.g*., IL-2, IL-15), or through the use of genetically modified antigen-presenting and/or stimulatory cell lines which display the antigen targeted by the CAR binding domain (and in some cases additional costimulatory molecules), or in some embodiments, which express a membrane-bound form of a cytokine, such as for example interleukin (IL)-15 and 41BB ligand (K562-mb15-41BBL cell line; Fujisaki 2009, Cancer Res. 59:4010-4017). Other methods to selectively propagate NK cells to constitutively express CAR include co-expression with transgenes for selection under cytocidal concentrations of drug and/or sorting, such as using magnetic beads that recognize introduced proteins co-expressed with CAR. Activated CAR-NK cells (*e.g*., expanded and activated CAR-NK cells) may be referred to herein as "CAR-NK cell-derived effector cells".

Prior to administration, and following expansion, the CAR-NK cells are treated with a nucleic acid targeting agent, such as for example, an agent that induces adduct formation, a cross-linking agent, a psoralen, a nitrogen mustard, cis-platin, a bulky adduct, ultraviolet light, gamma irradiation, any combination thereof, and the like. Typically, the lesion produced by one molecule of cross-linking agent involves cross-linking of both strands of the double helix, and most preferably the creation of interstrand crosslinks in the DNA helix. Treatment of cells with a nucleic acid targeting agent, such as a light sensitive nucleic acid cross-linking agent (*e.g*., photochemical agent, a psoralen followed by exposure to ultraviolet light) is described in U.S. Patents 5,399,719 and 5,593,823. Because some degree of function and/or cytokine release is necessary for CAR-NK cell-based therapy, adducts and/or crosslinks are introduced in an amount necessary to prevent cell division (and so NK cell proliferation), but that permits the CAR-NK cell-derived effector cells to retain sufficient function, such as for example, the expression of effector cytokines. The use of such agents generally results in a proliferation incompetent CAR-NK cell-derived effector cell population that maintains desired function (*e.g*., immune function, cytolytic function) and/or immunological activity, including for example, the ability of the effector cell population to promote destruction of a diseased cell.

In a preferred embodiment, the nucleic acid targeting agent is present in an amount effective to form from about 10² to about 10⁴ adducts per 10⁸ base pairs of genomic DNA of the leukocytes. Preferably, the method results in proliferation being inhibited in at least 90% of the CAR-T-derived effector cells. Suitable nucleic acid targeting agents comprise an alkylator selected from the group consisting of mustards, mustard intermediates and mustard equivalents; a nucleic acid targeting group selected from the group consisting of intercalators, minor groove binders, major groove binders, electrostatic binders, and sequence-specific binders; frangible anchor linker effector (FRALE) compounds (see *e.g.*, U.S. Patents 6,093,725 and 6,514,987) such as β-alanine; N-(acridin-9-yl), 2-[bis(2-chloroethyl)amino]ethyl ester; or a photoactivatable moiety selected from the group consisting of furocoumarins, actinomycins, anthracyclinones, anthramycins, benzodipyrones, fluorenes, fluorenones, monostral fats blue, norphillin A, organic dyes; phenanthridines, phenazathionium salts, phenazines, phenothiazines, phenylazides, quinolines and thiaxanthenones acridines and ellipticenes, or a combination thereof. Preferred are psoralen and psoralen-derived compounds activated by UVA irradiation (see *e.g.*, U.S. Patents 5,399,719 and 5,593,823). In a preferred embodiment, the psoralen is 4'-(4-amino-2-oxa)butyl-4,5',8-trimethylpsoralen, 4'aminomethyl 4, 5', 8-trimethylpsoralen (AMT), 5-methoxy psoralen, trioxalen 4, 5', 8-trimethylpsoralen, or 8-methoxy psoralen. This list is not meant to be limiting.

The number of covalent adducts that the compound forms in the CAR-T-derived effector cell nucleic acid can be modulated so that the compound inhibits proliferation but maintains immune functions effective to promote destruction of a diseased cell. With alkylator compounds, the effect can be modulated by adjusting the concentration of the compound and the length of time the cells are contacted with the compound before removing unbound compound, as well as the addition of quenchers with different specificities. The concentration required will depend on the characteristics of the particular compound, such as its solubility in aqueous solution and the DNA binding constant. The compound will typically be used at concentrations effective to generate about 1 to 10⁴ adducts of the covalent binding compound per 10⁸ base pairs of leukocyte genomic DNA, preferably about 5 to 10³ adducts, even more preferably, about 10² to 10³ adducts. Ideally, the conditions for treating the cell population will generate about 10³ adducts per 10⁸ base pairs of genomic DNA. The lowest concentration of compound effective to achieve the NK cell compositions of the present disclosure is the preferred concentration. The number of adducts can be modulated by the presence of certain additives in the compositions being treated. For example, the use of human serum albumin (HSA) in the illumination mixture, to help stabilize NK-cells during exposure to ultraviolet light, modifies the exposure dose required for a given level of reaction. In the case of HSA, a quenching effect is obtained, such that a higher exposure dose (*e.g*., higher compound concentration and/or higher light dose) is requried for equivalent level of reaction. The use of additives, such as HSA, in the photochemical treatment reaction provides greater flexibility in defining conditions for achieving reproducible levels of reaction using higher compound concentrations and/or light doses. As another example, glutathione or other thiol compounds can be used in addition to the alkylator compounds to modify their reactivity in different comaprtments of the cellular milieu, as well as the effective concentration of the compound reacting with nucleic acids to form adducts and crosslinks. It is expected that different thiol compounds will have different partitioning between the extra and intracellular domains. The choice of the thiol as well as the concentration can help modulate the number of adducts formed.

Where the compound used to treat the NK cells is psoralen, the psoralen can be present at a concentration in the range of 10⁻⁴ µM to 150 µM, more preferably 10⁻³ µM to 15µM, still more preferably 10⁻³ µM to 1.5µM; and the sample of NK cells is exposed to ultraviolet light having a wavelength in the range of 200 to 450 nm, preferably between 320 and 400 nm. Preferably, the ultraviolet light is provided at a dosage of between 10⁻³ to 100 J/cm², more preferably, 0.1 to 10 J/cm². The sample of NK cells will be exposed to the ultraviolet light for a period of 1 second to 60 minutes, preferably 6 seconds to 10 minutes. The sample of NK cells may be provided at a cell density of 10 to 10⁹ cells per mL, more preferably between 10² and 10⁸ cells pre mL, most preferably at 2 × 10⁶ cells per mL. In some embodiments, the sample may contain, in addition to 15nM psoralen, approximately 2 × 10⁶ cells/mL in 200 mL of solution also containing approximately 1% human serum albumin (HSA), 133 (g/ml sodium caprylate, 197 (g/ml sodium acetyltryptophanate, and 0.9% NaCl. The number of compound-DNA adducts resulting from treatment can be measured by methods known to those of skill in the art, for example, by using radiolabeled DNA binding compound as described in the Examples below, or by PCR analysis of long (*e.g*., 15-20 kilobase) amplicons (measuring the degree of inactivation of the amplicons) with statistical analysis of the results, as described by Yakes et al. (1997) Proc. Natl. Acad. Sci. USA 94:514-519.

The effect of treatment with the compound on the viability and function of the NK cell population can be monitored by *in vitro* as well as *in vivo* assays to determine the optimum treatment conditions that minimize proliferation of the CAR-NK cell-derived effector cells and other undesirable properties, such a for example, cytokine release syndrome activity, while maintaining cytotoxic function. While some functions and/or cytokine production by the the CAR-NK-derived effector cells may be inhibited, preferably such functions and/or cytokine production is maintained at least 70%, more preferably at 80%, even more preferably greater than 90%, and most preferably at least greater than 95%, of the level before treatment with the aforementioned compound. The presence of antigenic markers, CD56 (*e.g*., CD56^{bright}, CD56^{dim}), CD16, CD27 and NKp46, which are known to be involved in interactions associated with NK cell activation and immune function, can be determined as a function of concentration of the nucleic acid targeting agent. The effectiveness of a NK population to promote destruction of a diseased cell or pathogen can be measured by various assays, such as by MLR or by ⁵¹Cr release assay as described below in the Examples. Cytolytic effectiveness is demonstrated, *e.g*., by the ability to mediate killing of a leukemic cell in a ⁵¹Cr release assay.

For the purposes of this disclosure, a CAR-NK cell-derived effector cell population is considered effective to promote destruction of a diseased cell if, in an appropriate assay (*e.g*., the ⁵¹Cr release assay), the population exhibits cytolytic activity at a level of at least about 20% above that of the negative control population. In certain embodiments, the CAR-NK cell-derived effector cell population may exhibit cytolytic activity (*e.g*., in an appropriate assay) at a level of at least about 50%, at least about 60%, at least about 70%, at least about 80%, at least about 90%, at least about 95% or more above that of the negative control population. The diseased cell can be from any type of cancer, of any tissue or cell type origin. Suitable target cells include but are not limited to cells of the following malignancies: Leukemia including Chronic Myelogenous Leukemia (CML), Chronic Lymphocytic leukemia (CLL), Acute Myelogenous Leukemia (AML), and Acute Lymphoblastic Leukemia (ALL); Multiple myeloma (MM); Non-Hodgkin lymphoma and Hodgkin's disease (lymphoma); solid tumors, including breast, lung, ovarian and testicular cancers, prostate cancer, colon cancer, melanoma, renal carcinoma cell, neuroblastoma, and head and neck tumors.

### 3. Target Cancer Antigens

The CARs and CAR-NK and CAR-T-derived effector cell populations of the present disclosure preferably target one or more cancer antigens. Examples of target antigens that may find use in the disclosure are listed in the following table and for example, in Glienke et al. (2015, Front. Pharmacol. 6:1-7). The target antigen may also be a fragment or fusion polypeptide comprising an immunologically active portion of the antigens listed in the table. This list is not meant to be limiting.

**Table 1. Antigens.**

| **ANTIGEN** | **REFERENCE** |
|---|---|
| **Tumor antigens** | |
| CD19 | GenBank Acc. No. NM_001178098; NP_001171569; UniProt P15391 (The Lancet, Early Online Publication, 13 October 2014 doi:10.1016/S0140-6736(14)61403-3). |
| Mesothelin | GenBank Acc. No. NM_005823; U40434; NM_013404; BC003512 (see also, e.g., Hassan, et al. (2004) Clin. Cancer Res. 10:3937-3942; Muminova, et al. (2004) BMC Cancer 4:19; Iacobuzio-Donahue, et al. (2003) Cancer Res. 63:8614-8622). |
| Wilms' tumor-1 associated protein (Wt-1), including isoform A; isoform B; isoform C; isoform D. | WT-1 isoform A (GenBank Acc. Nos. NM_000378; NP_000369). |
| | WT-1 isoform B (GenBank Acc. Nos. NM_024424; NP_077742). |
| | WT-1 isoform C (GenBank Acc. Nos. NM_024425; NP_077743). |
| | WT-1 isoform D (GenBank Acc. Nos. NM_024426; NP_077744). |
| Stratum corneum chymotryptic enzyme (SCCE), and variants thereof. | GenBank Acc. No. NM_005046; NM_139277; AF332583. See also, e.g., Bondurant, et al. (2005) Clin. Cancer Res. 11:3446-3454; Santin, et al. (2004) Gynecol. Oncol. 94:283-288; Shigemasa, et al. (2001) Int. J. Gynecol. Cancer 11:454-461; Sepehr, et al. (2001) Oncogene 20:7368-7374. |
| MHC class I chain-related protein A (MICA); MHC class I chain-related protein A (MICB). | See, e.g., Groh, et al. (2005) Proc. Natl. Acad. Sci. USA 102:6461-6466; GenBank Acc. Nos. NM_000247; BC_016929; AY750850; NM_005931. |
| Gastrin and peptides derived from gastrin; gastrin/CCK-2 receptor (also known as CCK-B). | Harris, et al. (2004) Cancer Res. 64:5624-5631; Gilliam, et al. (2004) Eur. J. Surg. Oncol. 30:536-543; Laheru and Jaffee (2005) Nature Reviews Cancer 5:459-467. |
| Glypican-3 (an antigen of, e.g., hepatocellular carcinoma and melanoma). | GenBank Acc. No. NM_004484 Nakatsura, et al. (2003) Biochem. Biophys. Res. Commun. 306:16-25; Capurro, et al. (2003) Gasteroenterol. 125:89-97; Nakatsura, et al. (2004) Clin. Cancer Res. 10:6612-6621). |
| Coactosin-like protein. | Nakatsura, et al. (2002) Eur. J. Immunol. 32:826-836; Laheru and Jaffee (2005) Nature Reviews Cancer 5:459-467. |
| Prostate stem cell antigen (PSCA). | GenBank Acc. No. AF043498; AR026974; AR302232 (see also, e.g., Argani, et al. (2001) Cancer Res. 61:4320-4324; Christiansen, et al. (2003) Prostate 55:9-19; Fuessel, *et al.* (2003) 23:221-228). |
| Prostate acid phosphatase (PAP); prostate-specific antigen (PSA); PSM; PSMA. | Small, et al. (2000) J. Clin. Oncol. 18:3894-3903; Altwein and Luboldt (1999) Urol. Int. 63:62-71; Chan, et al. (1999) Prostate 41:99-109; Ito, et al. (2005) Cancer 103:242-250; Schmittgen, et al. (2003) Int. J. Cancer 107:323-329; Millon, et al. (1999) Eur. Urol. 36:278-285. |
| Six-transmembrane epithelial antigen of prostate (STEAP). | See, e.g., Machlenkin, et al. (2005) Cancer Res. 65:6435-6442; GenBank Acc. No. NM_018234; NM_001008410; NM_182915; NM_024636; NM_012449; BC011802. |
| Prostate carcinoma tumor antigen-1 (PCTA-1). | See, e.g., Machlenkin, et al. (2005) Cancer Res. 65:6435-6442; GenBank Acc. No. L78132. |
| Prostate tumor-inducing gene-1 (PTI-1). | See, e.g., Machlenkin, et al. (2005) Cancer Res. 65:6435-6442). |
| Prostate-specific gene with homology to G protein-coupled receptor. | See, e.g., Machlenkin, et al. (2005) Cancer Res. 65:6435-6442). |
| Prostase (an antrogen regulated serine protease). | See, e.g., Machlenkin, et al. (2005) Cancer Res. 65:6435-6442; GenBank Acc. No. BC096178; BC096176; BC096175. |
| Proteinase 3. | GenBank Acc. No. X55668. |
| Cancer-testis antigens, e.g., NY-ESO-1; SCP-1; SSX-1; SSX-2; SSX-4; GAGE, CT7; CT8; CT10; MAGE-1; MAGE-2; MAGE-3; MAGE-4; MAGE-6; LAGE-1. | GenBank Acc. No. NM_001327 (NY-ESO-1) (see also, e.g., Li, et al. (2005) Clin. Cancer Res. 11:1809-1814; Chen, et al. (2004) Proc. Natl. Acad. Sci. USA. 101(25):9363-9368; Kubuschok, et al. (2004) Int. J. Cancer. 109:568-575; Scanlan, et al. (2004) Cancer Immun. 4:1; Scanlan, et al. (2002) Cancer Res. 62:4041-4047; Scanlan, et al. (2000) Cancer Lett. 150:155-164; Dalerba, et al. (2001) Int. J. Cancer 93:85-90; Ries, et al. (2005) Int. J. Oncol. 26:817-824. |
| MAGE-A1, MAGE-A2; MAGE-A3; MAGE-A4; MAGE-A6; MAGE-A9; MAGE-A10; MAGE-A12; GAGE-3/6; NT-SAR-35; BAGE; CA125. | Otte, et al. (2001) Cancer Res. 61:6682-6687; Lee, et al. (2003) Proc. Natl. Acad. Sci. USA 100:2651-2656; Sarcevic, et al. (2003) Oncology 64:443-449; Lin, et al. (2004) Clin. Cancer Res. 10:5708-5716. |
| GAGE-1; GAGE-2; GAGE-3; GAGE-4; GAGE-5; GAGE-6; GAGE-7; GAGE-8; GAGE-65; GAGE-11; GAGE-13; GAGE-7B. | De Backer, et al. (1999) Cancer Res. 59:3157-3165; Scarcella, et al. (1999) Clin. Cancer Res. 5:335-341. |
| HIP1R; LMNA; KIAA1416; Seb4D; KNSL6; TRIP4; MBD2; HCAC5; MAGEA3. | Scanlan, et al. (2002) Cancer Res. 62:4041-4047. |
| DAM family of genes, e.g., DAM-1; DAM-6. | Fleishhauer, et al. (1998) Cancer Res. 58:2969-2972. |
| RCAS1. | Enjoji, et al. (2004) Dig. Dis. Sci. 49:1654-1656. |
| RU2. | Van Den Eynde, et al. (1999) J. Exp. Med. 190:1793-1800. |
| CAMEL. | Slager, et al. (2004) J. Immunol. 172:5095-5102; Slager, et al. (2004) Cancer Gene Ther. 11:227-236. |
| Colon cancer associated antigens, e.g., NY-CO-8; NY-CO-9; NY-CO-13; | Scanlan, et al. (2002) Cancer Res. 62:4041-4047. |
| NY-CO-16; NY-CO-20; NY-CO-38; NY-CO-45; NY-CO-9/HDAC5; NY-CO-41/MBD2; NY-CO-42/TRIP4; NY-CO-95/KIAA1416; KNSL6; seb4D. | |
| N-Acetylglucosaminyl-tranferase V (GnT-V). | Dosaka-Akita, et al. (2004) Clin. Cancer Res. 10:1773-1779. |
| Elongation factor 2 mutated (ELF2M). | Renkvist, et al. (2001) Cancer Immunol Immunother. 50:3-15. |
| HOM-MEL-40/SSX2 | Neumann, et al. (2004) Int. J. Cancer 112:661-668; Scanlan, et al. (2000) Cancer Lett. 150:155-164. |
| BRDT. | Scanlan, et al. (2000) Cancer Lett. 150:155-164. |
| SAGE; HAGE. | Sasaki, et al. (2003) Eur. J. Surg. Oncol. 29:900-903. |
| RAGE. | See, e.g., Li, et al. (2004) Am. J. Pathol. 164:1389-1397; Shirasawa, et al. (2004) Genes to Cells 9:165-174. |
| MUM-1 (melanoma ubiquitous mutated); MUM-2; MUM-2 Arg-Gly mutation; MUM-3. | Gueguen, et al. (1998) J. Immunol. 160:6188-6194; Hirose, et al. (2005) Int. J. Hematol. 81:48-57; Baurain, et al. (2000) J. Immunol. 164:6057-6066; Chiari, et al. (1999) Cancer Res. 59:5785-5792. |
| LDLR/FUT fusion protein antigen of melanoma. | Wang, et al. (1999) J. Exp. Med. 189:1659-1667. |
| NY-REN series of renal cancer antigens. | Scanlan, et al. (2002) Cancer Res. 62:4041-4047; Scanlan, et al. (1999) Cancer Res. 83:456-464. |
| NY-BR series of breast cancer antigens, e.g., NY-BR-62; NY-BR-75; NY-BR-85; NY-BR-62; NY-BR-85. | Scanlan, et al. (2002) Cancer Res. 62:4041-4047; Scanlan, et al. (2001) Cancer Immunity 1:4. |
| BRCA-1; BRCA-2. | Stolier, et al. (2004) Breast J. 10:475-480; Nicoletto, et al. (2001) Cancer Treat Rev. 27:295-304. |
| DEK/CAN fusion protein | Von Lindern, et al. (1992) Mol. Cell. Biol. 12:1687-1697. |
| Ras, e.g., wild type ras, ras with mutations at codon 12, 13, 59, or 61, e.g., mutations G12C; G12D; G12R; G12S; G12V; G13D; A59T; Q61H. K-RAS; H-RAS; N-RAS. | GenBank Acc. Nos. P01112; P01116; M54969; M54968; P01111; P01112; K00654. See also, e.g., GenBank Acc. Nos. M26261; M34904; K01519; K01520; BC006499; NM_006270; NM_002890; NM_004985; NM_033360; NM_176795; NM_005343. |
| BRAF (an isoform of RAF). | Tannapfel, et al. (2005) Am. J. Clin. Pathol. 123:256-2601; Tsao and Sober (2005) Dermatol. Clin. 23:323-333. |
| Melanoma antigens, including HST-2 melanoma cell antigens. | GenBank Acc. No. NM_206956; NM_206955; NM_206954; NM_206953; NM_006115; NM_005367; NM_004988; AY148486; U10340; U10339; M77481. See, e g., Suzuki, et al. (1999) J. Immunol. 163:2783-2791. |
| Survivin | GenBank Acc. No. AB028869; U75285 (see also, e.g., Tsuruma, et al. (2004) J. Translational Med. 2:19 (11 pages); Pisarev, et al. (2003) Clin. Cancer Res. 9:6523-6533; Siegel, et al. (2003) Br. J. Haematol. 122:911-914; Andersen, et al. (2002) Histol. Histopathol. 17:669-675). |
| MDM-2 | NM_002392; NM_00687 (see also, e.g., Mayo, et al. (1997) Cancer Res. 57:5013-5016; Demidenko and Blagosklonny (2004) Cancer Res. 64:3653-3660). |
| Methyl-CpG-binding proteins (MeCP2; MBD2). | Muller, et al. (2003) Br. J. Cancer 89:1934-1939; Fang, et al. (2004) World J. Gastreenterol. 10:3394-3398. |
| NA88-A. | Moreau-Aubry, et al. (2000) J. Exp. Med. 191:1617-1624. |
| Histone deacetylases (HDAC), e.g., HDAC5. | Waltregny, et al. (2004) Eur. J. Histochem. 48:273-290; Scanlan, et al. (2002) Cancer Res. 62:4041-4047. |
| Cyclophilin B (Cyp-B). | Tamura, et al. (2001) Jpn. J. Cancer Res. 92:762-767. |
| CA 15-3; CA 27.29. | Clinton, et al. (2003) Biomed. Sci. Instrum. 39:408-414. |
| Heat shock protein Hsp70. | Faure, et al. (2004) Int. J. Cancer 108:863-870. |
| GAGE/PAGE family, e.g., PAGE-1; PAGE-2; PAGE-3; PAGE-4; XAGE-1; XAGE-2; XAGE-3. | Brinkmann, et al. (1999) Cancer Res. 59:1445-1448. |
| MAGE-A, B, C, and D families. MAGE-B5; MAGE-B6; MAGE-C2; MAGE-C3; MAGE-3; MAGE-6. | Lucas, et al. (2000) Int. J. Cancer 87:55-60; Scanlan, et al. (2001) Cancer Immun. 1:4. |
| Kinesin 2; TATA element modulatory factor 1; tumor protein D53; NY | Scanlan, et al. (2001) Cancer Immun. 30:1-4. |
| Alpha-fetoprotein (AFP) | Grimm, et al. (2000) Gastroenterol. 119:1104-1112. |
| SART1; SART2; SART3; ART4. | Kumamuru, et al. (2004) Int. J. Cancer 108:686-695; Sasatomi, et al. (2002) Cancer 94:1636-1641; Matsumoto, et al. (1998) Jpn. J. Cancer Res. 89:1292-1295; Tanaka, et al. (2000) Jpn. J. Cancer Res. 91:1177-1184. |
| Preferentially expressed antigen of melanoma (PRAME). | Matsushita, et al. (2003) Leuk. Lymphoma 44:439-444; Oberthuer, et al. (2004) Clin. Cancer Res. 10:4307-4313. |
| Carcinoembryonic antigen (CEA), CAP1-6D enhancer agonist peptide. | GenBank Acc. No. M29540; E03352; X98311; M17303 (see also, e.g., Zaremba (1997) Cancer Res. 57:4570-4577; Sarobe, et al. (2004) Curr. Cancer Drug Targets 4:443-454; Tsang, et al. (1997) Clin. Cancer Res. 3:2439-2449; Fong, et al. (2001) Proc. Natl. Acad. Sci. USA 98:8809-8814). |
| HER-2/neu. | Disis, et al. (2004) J. Clin. Immunol. 24:571-578; Disis and Cheever (1997) Adv. Cancer Res. 71:343-371. |
| Cdk4; cdk6; p16 (INK4); Rb protein. | Ghazizadeh, et al. (2005) Respiration 72:68-73; Ericson, et al. (2003) Mol. Cancer Res. 1:654-664. |
| TEL; AML1; TEL/AML1. | Stams, et al. (2005) Clin. Cancer Res. 11:2974-2980. |
| Telomerase (TERT). | Nair, et al. (2000) Nat. Med. 6:1011-1017. |
| 707-AP. | Takahashi, et al. (1997) Clin. Cancer Res. 3:1363-1370. |
| Annexin, e.g., Annexin II. | Zimmerman, et al. (2004) Virchows Arch. 445:368-374. |
| BCR/ABL; BCR/ABL p210; BCR/ABL p190; CML-66; CML-28. | Cobaldda, et al. (2000) Blood 95:1007-1013; Hakansson, et al. (2004) Leukemia 18:538-547; Schwartz, et al. (2003) Semin. Hematol. 40:87-96; Lim, et al. (1999) Int. J. Mol. Med. 4:665-667. |
| BCL2; BLC6; CD 10 protein. | Iqbal, et al. (2004) Am. J. Pathol. 165:159-166. |
| CDC27 (this is a melanoma antigen). | Wang, et al. (1999) Science 284:1351-1354. |
| Sperm protein 17 (SP17); 14-3-3-zeta; MEMD; KIAA0471; TC21. | Arora, et al. (2005) Mol. Carcinog. 42:97-108. |
| Tyrosinase-related proteins 1 and 2 (TRP-1 and TRP-2). | GenBank Acc. No. NM_001922. (see also, e.g., Bronte, et al. (2000) Cancer Res. 60:253-258). |
| Gp100/pmel-17. | GenBank Acc. Nos. AH003567; U31798; U31799;U31807; U31799 (see also, e.g., Bronte, et al. (2000) Cancer Res. 60:253-258). |
| TARP. | See, e.g., Clifton, et al. (2004) Proc. Natl. Acad. Sci. USA 101:10166-10171; Virok, et al. (2005) Infection Immunity 73:1939-1946. |
| Tyrosinase-related proteins 1 and 2 (TRP-1 and TRP-2). | GenBank Acc. No. NM_001922. (see also, e.g., Bronte, et al. (2000) Cancer Res. 60:253-258). |
| Melanocortin 1 receptor (MC1R); MAGE-3; gp100; tyrosinase; dopachrome tautomerase (TRP-2); MART-1. | Salazar-Onfray, et al. (1997) Cancer Res. 57:4348-4355; Reynolds, et al. (1998) J. Immunol. 161:6970-6976; Chang, et al. (2002) Clin. Cancer Res. 8:1021-1032. |
| MUC-1; MUC-2. | See, e.g., Davies, et al. (1994) Cancer Lett. 82:179-184; Gambus, et al. (1995) Int. J. Cancer 60:146-148; McCool, et al. (1999) Biochem. J. 341:593-600. |
| Spas-1. | U.S. Published Pat. Appl. No. 20020150588 of Allison, et al. |
| CASP-8; FLICE; MACH. | Mandruzzato, et al. (1997) J. Exp. Med. 186:785-793. |
| CEACAM6; CAP-1. | Duxbury, et al. (2004) Biochem. Biophys. Res. Commun. 317:837-843; Morse, et al. (1999) Clin. Cancer Res. 5:1331-1338. |
| HMGB1 (a DNA binding protein and cytokine). | Brezniceanu, et al. (2003) FASEB J. 17:1295-1297. |
| ETV6/AML1. | Codrington, et al. (2000) Br. J. Haematol. 111:1071-1079. |
| Mutant and wild type forms of adenomatous polyposis coli (APC); beta-catenin; c-met; p53; E-cadherin; cyclooxygenase-2 (COX-2). | Clements, et al. (2003) Clin. Colorectal Cancer 3:113-120; Gulmann, et al. (2003) Appl. Immunohistochem. Mol. Morphol. 11:230-237; Jungck, et al. (2004) Int. J. Colorectal. Dis. 19:438-445; Wang, et al. (2004) J. Surg. Res. 120:242-248; Abutaily, et al. (2003) J. Pathol. 201:355-362; Liang, et al. (2004) Br. J. Surg. 91:355-361; Shirakawa, et al. (2004) Clin. Cancer Res. 10:4342-4348. |
| Renal cell carcinoma antigen bound by mAB G250. | Mulders, et al. (2003) Urol. Clin. North Am. 30:455-465; Steffens, et al. (1999) Anticancer Res. 19:1197-1200. |
| EphA2 | See, e.g., U.S. Patent Publication No. 2005/0281783 A1; Genbank Accession No. NM_004431 (human); Genbank Accession No. NM_010139 (Mouse); Genbank Accession No. AB038986 (Chicken, partial sequence); GenBank Accession Nos. NP_004422, AAH37166, and AAA53375 (human); GenBank Accession Nos. NP_034269 (mouse), AAH06954 (mouse), XP_345597 (rat), and BAB63910 (chicken). |
| EGFRvIII | See, e.g., WO/2012/068360 |

### 4. Therapeutic compositions.

The cell compositions described herein can be administered to a host, either alone or in combination with a pharmaceutically acceptable excipient, in an amount sufficient to induce an appropriate immune (*e.g*., anti-tumor, anti-viral) response. The response can comprise, without limitation, specific immune response, non-specific immune response, both specific and non-specific response, innate response, primary immune response, adaptive immunity, secondary immune response, memory immune response, immune cell activation, immune cell proliferation, immune cell differentiation, and cytokine expression.

The disclosure provides a method of providing an anti-tumor immunity in a mammal by administering to a mammal an effective amount of a cell genetically modified to express a CAR. An "effective amount" as used herein means an amount which provides a therapeutic or prophylactic benefit. Effective amounts of CAR-NK cells can be determined by a physician with consideration of individual differences in age, weight, tumor size, extent of infection or metastasis, and condition of the patient (subject). It can generally be stated that a pharmaceutical composition comprising the CAR-NK cells described herein may be administered at a dosage of 10⁴ to 10¹¹ cells/kg body weight, preferably 10⁷ to 10¹⁰ cells/kg body weight, including all integer values within those ranges. NK cell compositions may also be administered multiple times at these dosages. The cells can be administered by using infusion techniques that are commonly known in immunotherapy (see, *e.g*., Rosenberg et al, New Eng. J. of Med. 319: 1676, 1988). The optimal dosage and treatment regime for a particular patient can readily be determined by one skilled in the art of medicine by monitoring the patient for signs of disease and adjusting the treatment accordingly.

An effective amount of the cell compositions described herein may be given in one dose, but is not restricted to one dose. Thus, the administration can be two, three, four, five, six, seven, eight, nine, ten, eleven, twelve, thirteen, fourteen, fifteen, sixteen, seventeen, eighteen, nineteen, twenty, or more, administrations of the cells. Where there is more than one administration in the present methods, the administrations can be spaced by time intervals of one minute, two minutes, three, four, five, six, seven, eight, nine, ten, or more minutes, by intervals of about one hour, two hours, three, four, five, six, seven, eight, nine, ten, 11, 12, 13, 14, 15, 16, 17, 18, 19, 20, 21, 22, 23, 24 hours, and so on. In the context of hours, the term "about" means plus or minus any time interval within 30 minutes. The administrations can also be spaced by time intervals of one day, two days, three days, four days, five days, six days, seven days, eight days, nine days, ten days, 11 days, 12 days, 13 days, 14 days, 15 days, 16 days, 17 days, 18 days, 19 days, 20 days, 21 days, and combinations thereof. The disclosure is not limited to dosing intervals that are spaced equally in time, but encompass doses at non-equal intervals, such as a priming schedule consisting of administration at 1 day, 4 days, 7 days, and 25 days, just to provide a non-limiting example.

A "pharmaceutically acceptable excipient" or "diagnostically acceptable excipient" includes but is not limited to, sterile distilled water, saline, phosphate buffered solutions, amino acid based buffers, or bicarbonate buffered solutions. An excipient selected and the amount of excipient used will depend upon the mode of administration. Administration comprises an injection, infusion, or a combination thereof.

An effective amount for a particular patient may vary depending on factors such as the condition being treated, the overall health of the patient, the route and dose of administration and the severity of side effects. Guidance for methods of treatment and diagnosis is available (see, e.g., Maynard, et al. (1996) A Handbook of SOPs for Good Clinical Practice, Interpharm Press, Boca Raton, FL; Dent (2001) Good Laboratory and Good Clinical Practice, Urch Publ., London, UK).

The cell compositions of the present disclosure can be administered in a dose, or dosages, where each dose comprises at least 100 cells/kg body weight or more; in certain embodiments 1000 cells/kg body weight or more; normally at least 10,000 cells; more normally at least 100,000 cells; most normally at least 1 million cells; often at least 10 million cells; more often at least 100 million cells; typically at least 1 billion cells; usually at least 10 billion cells; conventionally at least 100 billion cells; and sometimes at least 1 trillion cells/kg body weight.

A dosing schedule of, for example, once/week, twice/week, three times/week, four times/week, five times/week, six times/week, seven times/week, once every two weeks, once every three weeks, once every four weeks, once every five weeks, and the like, is provided. The dosing schedules encompass dosing for a total period of time of, for example, one week, two weeks, three weeks, four weeks, five weeks, six weeks, two months, three months, four months, five months, six months, seven months, eight months, nine months, ten months, eleven months, and twelve months.

Provided are cycles of the above dosing schedules. The cycle can be repeated about, e.g., every seven days; every 14 days; every 21 days; every 28 days; every 35 days; 42 days; every 49 days; every 56 days; every 63 days; every 70 days; and the like. An interval of non dosing can occur between a cycle, where the interval can be about, e.g., seven days; 14 days; 21 days; 28 days; 35 days; 42 days; 49 days; 56 days; 63 days; 70 days; and the like. In this context, the term "about" means plus or minus one day, plus or minus two days, plus or minus three days, plus or minus four days, plus or minus five days, plus or minus six days, or plus or minus seven days.

Methods for co-administration with an additional therapeutic agent are well known in the art (Hardman, et al. (eds.) (2001) Goodman and Gilman's The Pharmacological Basis of Therapeutics, 10th ed., McGraw-Hill, New York, NY; Poole and Peterson (eds.) (2001) Pharmacotherapeutics for Advanced Practice:A Practical Approach, Lippincott, Williams & Wilkins, Phila., PA; Chabner and Longo (eds.) (2001) Cancer Chemotherapy and Biotherapy, Lippincott, Williams & Wilkins, Phila., PA).

Co-administration need not refer to administration at the same time in an individual, but rather may include administrations that are spaced by hours or even days, weeks, or longer, as long as the administration of multiple therapeutic agents is the result of a single treatment plan. By way of example, the CAR-NK effector cells of the present disclosure may be co-administered with CAR-NK cells having the same chimeric receptor, but which are not attenuated for proliferation as described herein. The co-administration may comprise administering the CAR-NK effector cells of the present disclosure before, after, or at the same time as the "traditional" CAR-NK cells. In one exemplary treatment schedule, the CAR-NK effector cells of the present disclosure may be given as an initial dose in a multi-day protocol, with "traditional" CAR-NK cells given on later administration days; or the "traditional" CAR-NK cells given as an initial dose in a multi-day protocol, with the CAR-NK effector cells of the present disclosure given on later administration days. Alternatively, "traditional" CAR-NK cells and the CAR-NK effector cells of the present disclosure may be administered on alternate days in a mult-day protocol. In still another alternative, a mixture of "traditional" CAR-NK cells and the CAR-NK effector cells of the present disclosure may be administered to reduce the number of proliferation-competent cells in a single administration while maintaining an effective NK cell dose. This is not meant to be a limiting list of possible administration protocols. Alternatively, or in addition, CAR-NK cells of the present disclosure may be co-administered with CAR-T cells having the same chimeric receptor, or a different chimeric receptor. Such CAR-T cells may be "traditional" CAR-T cells, which are not attenuated for proliferation, or may be CAR-T cells which are attenuated for proliferation such as in a similar manner described herein for CAR-NK cells. Co-administration regimes with CAR-NK and CAR-T cells may include, for example, the aforementioned co-administration regimes described for CAR-NK cells and traditional CAR-NK cells.

Alternatively, or in addition, CAR-NK cells of the present disclosure may be co-administered with an additional therapeutic agent, such as a pharmaceutical or biological (*e.g*., antibody) agent suitable for the intended disease or condition. Antibodies may include for example, those which are specific for a single antigen (*e.g*., anti-KIR antibody, see for example, Benson, 2012, Blood, 120:4324-4333), as well as bi- or multispecific antibodies, (*e.g*.,BiKE and TriKE antibodies, see for example, Gleason et al, 2012, Mol. Cancer Ther. 11:2674-2684; Miller, 2013, ASH Education Book, Hematology 2013:247-253).

An effective amount of a therapeutic agent is one that will decrease or ameliorate the symptoms normally by at least 10%, more normally by at least 20%, most normally by at least 30%, typically by at least 40%, more typically by at least 50%, most typically by at least 60%, often by at least 70%, more often by at least 80%, and most often by at least 90%, conventionally by at least 95%, more conventionally by at least 99%, and most conventionally by at least 99.9%.

Formulations of therapeutic agents may be prepared for storage by mixing with physiologically acceptable carriers, excipients, or stabilizers in the form of, e.g., lyophilized powders, slurries, aqueous solutions or suspensions (see, e.g., Hardman, et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY).

### EXAMPLES

### Example 1. Preparation of CAR-NK cells attenuated for proliferation from PBMC

Cytokine induced killer cells are generated from PBMC against the receptor IL3RA (CD123) for treatment of AML as described in (Tettamanti et al., 2013, Brit. J. Haematol. 161:389-401). PBMCs of healthy subjects are obtained after centrifugation of fresh blood on a density gradient using Ficoll-Hypaque (Pharmacia LKB, Uppsala, Sweden). Cells are then resuspended in complete Advanced RPMI medium (Invitrogen). At the beginning of the culture, gamma interferon (IFN-g) is added at 1000 U/ml. The next day, IL-2 (Chiron B.V, Emeryville, CA, USA) and OKT3 (Janssen-Cilag S.p.A., Cologne Monzese, Italy) are added at 300 U/ml and at 50 ng/ml, respectively, and cells are kept at the initial concentration of 3×10⁶ cells/ml. Cells are then cultured for 21 days. Fresh medium and IL-2 are added weekly during culture and cell concentration is maintained at around 0.5×10⁶cells/ml.

Cloning of the anti-CD123.CAR, retroviral supernatant production and CIK cells transduction. The scFv for CD123 is generated starting from the DNA amplification of the variable regions of the light chain (VL) and heavy chain (VH) of the mAb 7G3 (Sun et al, 1996), separated by a DNA sequence encoding for a linker region (Ser-Gly4)3 to allow proper orientation of the two variable units. The scFv-CD123 is cloned in frame with CH2CH3-CD28 transmembrane-z in the SFG retroviral construct. Retroviral supernatant is produced by TransIT-2020 (Mirus BIO, Madison, WI, USA) mediated co-transfection of 293T cells with the MoMLV gag-pol expression plasmid pEQPAM3(- E), the RD114 env expression plasmid pRDF and the SFG-anti-CD123.CAR vector. Supernatants containing retroviral particles are harvested 48 and 72 h after transfection, immediately frozen in dry ice, and stored at 80°C until further use. 293T cells are used to titrate virus concentration. For transduction, 0.5×10⁶ CIK cells at day 5 of culture are resuspended in 2.5 ml of thawed viral supernatant supplemented with IL-2 (600 U/ml), seeded on Retro- Nectin (TaKaRa BioEurope, Gennevilliers, France)-coated 14 ml tubes and incubated for 24 h in a humidified incubator at 37°C, 5% CO2. The day after, the viral supernatant is removed and CIK cells are resuspended in 2.5 ml of newly thawed viral supernatant supplemented with IL-2 (600 U/ml), and incubated for 72 h in a humidified incubator at 37°C, 5% CO2.

S-59 adduct frequency is determined by using ¹⁴C labeled S-59 psoralen for photochemical inactivation. After photochemical treatment using the radioactively labeled S-59, genomic DNA is isolated form the NK cells. The adduct frequency (defined as the base-pair interval between psoralen adducts) is determined by dividing the calculated DNA genomes by the S-59 psoralen molecules, determined from the DNA concentration, the radioactivity of each sample, and specific activity of ¹⁴C S-59 psoralen. These cells are then treated with an optimized dose of UVA and concentration range of S-59 (U.S. Patents 5,399,719 and 5,593,823) to optimize cell function and attenuation of proliferation, generating attenuated NK-CAR cells. Short and long term co-culture experiments to determine the CAR-NK cytotoxic activity vs NK cells and after S-59/UVA treatment, colony forming experiments and cytokine release assay to determine the CAR-NK ability to release cytokines vs. NK cells and after S-59/UVA treatment, are performed as described in Tettamantti et al. The cells generated in this fashion are then used to treat AML patients in a manner analogous to the one described below for advanced cancer.

### Example 2. Preparation of CAR-NK cells attenuated for proliferation from NK-92

The NK-92 cell line is engineered by lentiviral transduction to contain CD19 and CD 20 directed CAR, as described in Boissel et al. (2009, Leuk. Res. 33:1255-1259). NK-92 cells are maintained in Myelocult^{®} medium (StemCell Technologies) supplemented with 500 IU/mL Proleukin (recombinant human interleukin-2; Novartis Corporation). Daudi (Burkitt's lymphoma, CD19+CD20+, NK-92 cell-sensitive) and SUPB15 (BCR-ABL1+ B-precursor ALL, CD19+CD20-, NK-92 cell-resistant) cell lines are purchased from American Type Culture Collection (ATCC). Daudi, SUP-B15 and TMD-5 cells are transduced with pFUW-Luc lentivirus and selected with puromycin (Sigma- Aldrich). Daudi cells, which are normally sensitive to the cytotoxic activity of NK and NK-92 cells, lose such sensitivity upon transfection with this construct, and are subsequently referred as DaudiNKR (NK resistant).

The CAR constructs used in this study consist of single chain variable fragments (scFvs) from a murine antibody specific for human CD19 or CD20 linked to the CD3ζ chain of the TCR complex (first-generation CAR). The cDNAs coding for CD19- and CD20-specific CARs are subcloned from the retroviral vector pLXSN14,15 into lentiviral vectors such as pCL20c-IRES-GFP. The CAR-coding constructs are transfected into HEK-293T packaging cells alongside helper plasmids, using the FugeneTM lipofection system (Roche). Culture supernatants are collected after 48 h, filtered (with 0.22 µm filters) and stored at -80°C. A high supernatant infectivity, such as a titer of > 10⁷ infectious units/mL is important. NK-92 cells are transduced with pCL20c lentiviral particles as previously described, (Boissel Let al. Leuk Lymphoma 2012; 53:958-65) using 2×10⁶ cells in 6-well plates mixed with the corresponding lentiviral supernatant and at a multiplicity of infection (MOI) of ~5. 15 µg/mL protamine sulfate (Sigma-Aldrich) is also used. Transduced cells are expanded in Myelocult^{®} medium supplemented with 1000 UI/mL Proleukin and GFP-expressing (*i.e*. transduced) NK-92 cells are further enriched by cell sorting to achieve > 95% purity (MoFlo, DakoCytomation). Transgene expression is confirmed by flow cytometry, measuring either mCherry or GFP expression directly, or CAR expression by using biotin-conjugated anti-scFv antibody (Jackson Immunoresearch) and allophycocyanin (APC)-conjugated streptavidin (BD Biosciences PharMingen).

S-59 adduct frequency is determined by using ¹⁴C labeled S-59 psoralen for photochemical inactivation. After photochemical treatment using the radioactively labeled S-59, genomic DNA is isolated form the NK cells. The adduct frequency (defined as the base-pair interval between psoralen adducts) is determined by dividing the calculated DNA genomes by the S-59 psoralen molecules, determined from the DNA concentration, the radioactivity of each sample, and specific activity of ¹⁴C S-59 psoralen. The cells are treated with an optimized dose of UVA and concentration range of S-59 to optimize function and inability to proliferate (attenuation), generating NK-CAR cells against CD19 or CD 20.

### Example 3. Xenotransplantion studies in mice

NOD.CB 17-Prkdcscid /J (NOD/SCID) and NOD.Cg-PrkdcscidIl2rgtm1Wjl /SzJ (NOD scid gamma, or NSG) mice are purchased from the Jackson Laboratory and housed under sterile conditions in an animal facility. For the Ph+ B-acute lymphoblastic leukemia model, 6- to 10-week old female NSG mice are sub-lethally irradiated with 350 cGy, 24 hours before the intravenous injection of 2×10⁵ or 1×10³ Luc-expressing SUP-B15 cells, or 5×10⁶ Lucexpressing TMD-5 cells (in PBS). Mice are then treated with 3 to 5 intravenous injections of 1×10⁷ parental NK-92 cells, or NK-92 cells expressing CD19- or CD20-targeting CARs, as well as the attenuated NK-92 CAR CD19 or CD 20 targeting cells every day or every 2 days as indicated. Intrafemoral injections are performed using 3×10⁶ NK-92 cells expressing CD19-specific CAR in PBS, or PBS alone (control), as previously described.

To test the local effect of CAR-expressing NK-92 cells, nonirradiated 6- to 10-week old female NOD/SCID mice are injected subcutaneously with 2.5×10⁵ Luc-expressing DaudiNKR cells in PBS. When the tumor has grown to about 0.5 cm³, mice are treated on days 4, 5 and 6 with intratumoral injections of 5×10⁶ parental NK-92 cells or NK-92 cells expressing CD19- or CD20-targeting CARs, in PBS.

### Example 4. Analysis of attenuated NK CAR cells in ADCC by cytotoxicity assay

Effector and target cells are co-cultured for 3 h and cytotoxicity is measured by flow cytometry. Briefly, CLL cells are incubated with rituximab, ofatumumab, or trastuzumab (as a negative control) for 30 min, followed by the addition of effector cells (parental NK-92 cells or NK-92Fc cells, which expressing high affinity FcyRIII), at an effector to target cell ratio of 10:1. Heat-inactivated FBS is utilized throughout the assays to exclude any contribution of complement-mediated cytotoxicity. For NK-mediated cytolysis, CLL target cells are incubated with parental NK-92 cells or NK-92 cells expressing CD20-specific CAR as above, in the absence of mAbs. Samples are analyzed immediately by flow cytometry. ADCC is calculated by subtracting the percentage of cytotoxicity obtained with mAbs plus parental NK-92 cells (which do not express FcyRIII) from that obtained with mAbs plus NK-92fc cells. The percentage of cytotoxicity obtained with mAbs or effector cells alone is expected to be lower than that detected with mAbs plus parental NK-92 cells. CAR-dependent killing is calculated by subtracting the percentage of target cell killing obtained with parental NK-92 cells from that obtained with CAR-expressing NK-92 cells and the effect of the S-59 treatment.

### Example 5. Infusion of NK-92 CAR cells

The activated NK cell line NK-92 (from Maki et al J Hematoth Stem Cell Res. 2001 10 369e83) is highly cytotoxic against a broad spectrum of malignant cells. The cells lack all currently known inhibitory KIR receptors, except for KIR2DL4, which is expressed at low levels, while expressing the full spectrum of activating receptors. The expansion kinetics of NK-92 have been delineated, and predictable numbers of activated NK-92 cells can be reproducibly obtained after culture in IL-2 (Maki et al 2001). The NK-92 cell line is a human IL-2-dependent NK cell line that was derived from a patient with non-Hodgkin's lymphoma. The cells are maintained in alpha medium supplemented with 2 mM L-glutamine, 0.2 mM I-inositol, 20 mM folic acid, 10-4 M 2-mercaptoethanol, 12.5% fetal calf serum (FCS), and 12.5% horse serum (Myelocult StemCell Technologies, Vancouver, BC, CA), in the presence of 100-200 IU/ml human IL-2 (Amgen, Thousand Oaks, CA or Biotest Pharma GmbH, Dreieich, Germany). These cells will then be treated with an optimized dose of UVA and concentration range of S-59 to optimize function and attenuation of proliferation, generating S-59 NK CAR cells.

Subjects with advanced cancer are treated with S-59 NK CAR cells, as described in Tonn et al. (2013, Cytotherapy 15:1563-1570). Patients are selected to have a negative cross-match with NK-92 cells. A commercial lymphocytotoxicity assay (One Lambda Inc, Canoga Park, CA, USA) is used. NK-92 cells are seeded at a density of 2.000 NK-92 cells and incubated with the patient's serum according to the manufacturer's instruction in the presence of rabbit complement. To detect dead cells, Fluoroquench is added. The reaction is considered negative if the number of dead cells is not increased compared with the negative control (NK-92 with anti-HLA anti ody negative control serum). For this phase I study, patients are treated at three different dose levels with two infusions of NK-92 cells 48 h apart at the following doses: 1×10⁹ cells/m2 (n=7) and 1×10¹⁰ cells/m2 (n =2). No lymphocyte-depleting chemotherapy is given before the NK cell infusion. A master cell bank of NK-92 cells is established prior to study start, with a total cell number of 5×10⁷ NK-92 kept in a 1-mL vial in 10% dimethyl sulfoxide, 40% heat-inactivated human plasma (German Red Cross Blood Donation Service, Baden-Würtemberg-Hessen,Germany) and 50% X-Vivo 10 medium (Lonza,Belgium). NK-92 cells are selected sterile and free of mycoplasma and human or animal-including porcine-pathogen viruses (Analysis Biomedical Test GmbH, Cologne, Germany). For the generation of individual cell dosages, a vial of the master cell bank of NK-92 cells is thawed and expanded in the presence of 1000 IU human recombinant IL-2 (Proleukin, Novartis,Basel, Switzerland) until cell growth is established. VuelLife culture bags (Cellgenix Technology GmbH, Freiburg, Germany) are prefilled with 3 L of X-Vivo 10 medium with 1000 IU IL-2 and 5% heat inactivated human plasma. NK-92 cells are added at a concentration of 2-5×10⁴ cells/mL. Samples for sterility testing are collected from each bag at the day of culture initiation. The expansion is performed as batch culture. The day before harvest, an additional 1000 IU/mL of Proleukin is added to the culture bags. Harvest of cells is performed in a closed system under class A conditions.

Briefly, the total volume of up to 20 L is transferred to 500-mL transfusion bags (Fresenius Bad Homburg, Germany) and centrifuged for 20 min at 2000 rpm. The supernatant is subsequently transferred to empty transfusion bags that are then connected to the transfusion bags before centrifugation with the use of a sterile connection device (T-SCD,Terumo, Eschborn, Germany). This process is repeated until all cell pellets are pooled into one 500-mL blood bag. Finally the NK-92 cells are spun down again and resuspended in 300 mL phosphate-buffered saline/ethylenediamine tetra-acetic acid buffer (Miltenyi Biotech, Cologne, Germany). Cell numbers are assessed with the use of a Sysmex XT 1800 automated cell analyzer. Cytokine production by NK-92 into the culture supernatant is determined after 24-h culture of the cells in X-Vivo 10 supplemented with 1000 IU/mL IL-2. A commercial cytokine bead array (Becton Dickinson, Heidelberg, Germany) is used to measure the following cytokines: IFN-g, TNF-a, IL-1b, IL-4, IL-5, IL-6, IL-8, IL-10 and IL-12p70. Before release, cell viability is determined by means of Trypan blue exclusion. Additionally, potency is assessed in a flow cytometric cytotoxicity assay (PKH-Assay) against K562. A further aliquot will be taken for sterility testing (BactAlert, Biomerieux, Nürtingen, Germany). NK-92 cell preparations are released if they fulfilled the following criteria: negative sterility at the time of batch culture initiation, viability >80% and cytotoxicity >50% at effector-to-target ratio of 10:1 against K562. Cells are irradiated with 10 Gy before intravenous infusion.

These cells are then treated with an optimized dose of UVA and concentration range of S-59 to optimize function and attenuation of proliferation, generating S-59 NK CAR cells. Before infusion, patients are started on hydration with normal saline at 250 mL/h for 2 hours. Patients are pre-medicated with methylprednisolone 2 mg/kg and a anti-histamine. The second scheduled infusion of cells 48 h later is given only if the first infusion is well tolerated and without adverse events grade >2. Regular blood work (complete blood cell count, electrolytes, creatinine and liver function tests) is performed weekly for 4 weeks after the first infusion. Briefly, 1 mg of genomic DNA extracted from peripheral blood is used in a single PCR reaction, and all experiments are performed in duplicate. Primers Y1 Forward (50-TCCAC TTTAT TCCAG GCCTG TCC-30, position 3511-3533 inYH10) andY2Revers (50-TTGAA TGGAA TGGGA ACGAA TGG-30, position 78-100 inYHl10), spanning the EcoRI restriction site of the tandem repeat target sequence pHY10, are synthesized by standards methods. To determine the lower threshold of sensitivity for detection of male DNA, mixtures of decreasing proportions of male DNA within excess female DNA were prepared, keeping the total amount of DNA constant. To document any immune response against the cells, HLA antibodies are determined by microcytotoxicity cross-match (One Lambda Flow- Pro, Canoga Park, CA, USA) at 1 and weeks after the last infusion. Serum levels of the following cytokines are measured at 0, 1, 4, 20, 24, 48 and 72 h after the first and second infusion using a commercially available cytokine bead array (Becton Dickinson): IFN-g, TNFa, IL-1b, IL-4, IL-5, IL-6, IL-8, IL-10 and IL-12p70. Patients have response assessments at 28 days after completing infusions. Responses are evaluated as complete response, partial response, stable disease and progressive disease, on the basis of Response Evaluation Criteria in Solid Tumors (RECIST) (26). Patients are not regularly scanned after 4 weeks but receive imaging studies when recurrence is suspected. The effect of NKCAR cells will be compared with NK CAR S-59 cells.

## Claims

1. A CAR natural killer (NK) cell-derived effector cell population comprising:
a population of NK cells expressing a chimeric antigen receptor (CAR), the CAR comprising an extracellular domain which specifically binds a predetermined antigen, a transmembrane domain, and a cytoplasmic co-stimulatory signaling domain, wherein the nucleic acid of the NK cells have been modified by reaction with a nucleic acid targeting compound, wherein the nucleic acid targeting compound is a psoralen or a nucleic acid alkylator, wherein the NK cells are present in the population in a therapeutically effective amount for treatment of a malignancy that expresses the predetermined antigen.

2. The CAR-NK cell-derived effector cell population of claim 1, wherein the population of NK cells expressing a chimeric antigen receptor comprises a population of activated NK cells expressing a chimeric antigen receptor.

3. The CAR-NK cell derived effector cell population of claim 1 or 2 wherein the reaction of the nucleic acid of the NK cells with the nucleic acid targeting compound results in interstrand cross-links and/or adducts in the nucleic acid and/or, wherein the nucleic acid of the NK cells have been modified by reaction with the nucleic acid targeting compound so that the NK cells are attenuated for proliferation.

4. The CAR-NK cell-derived effector cell population of any one of claims 1-3 wherein the nucleic acid alkylator is a FRALE (frangible anchor linker effector) such as β-alanine, N-(acridin-9-yl), 2-[bis(2-chloroethyl) amino] ethyl ester.

5. The CAR-NK cell-derived effector cell population of any one of claims 1-3 wherein the nucleic acid targeting compound is activated by illumination, wherein the nucleic acid targeting compound is preferably a psoralen compound activated by UVA illumination, wherein the psoralen is preferably 4'-(4-amino-2-oxa)butyl-4,5',8-trimethylpsoralen, 4'-aminomethyl-4,5',8-trimethylpsoralen (AMT), 5-methoxy psoralen, trioxalen, 4,5',8-trimethylpsoralen, or 8-methoxy psoralen.

6. The CAR-NK cell-derived effector cell population of claim 5 wherein the NK cells comprise psoralen-induced interstrand crosslinks introduced between the strands of the genomic DNA, wherein the interstrand crosslinks preferably inhibit replication of the NK cells.

7. The CAR-NK cell-derived effector cell population of any one of claims 1-6, wherein at least a portion of the NK cells produce one or more cytokines, wherein at least a portion of the NK cells produce one or more cytokines preferably selected from the group consisting of IFN-γ, GM-CSF, TNF and IL-10.

8. The CAR-NK cell-derived effector cell population of any one of claims 1-7, wherein at least a portion of the NK cells express one or more surface markers selected from the group consisting of CD56, CD16, CD27 and NKp46.

9. The CAR-NK cell-derived effector cell population of any one of claims 1-8 wherein greater than 90% of the NK cells in the population are non-proliferating.

10. The CAR-NK cell-derived effector cell population of any one of claims 2-8 wherein greater than 90% of the activated NK cells in the population are non-proliferating.

11. The CAR-NK cell-derived effector cell population of any one of claims 1-10, wherein the predetermined antigen is a cancer antigen, wherein the cancer is preferably selected from the group consisting of lung cancer, melanoma, breast cancer, prostate cancer, colon cancer, renal cell carcinoma, ovarian cancer, neuroblastoma, rhabdomyosarcoma, leukemia and lymphoma.

12. A CAR natural killer (NK) cell-derived effector cell population of any one of the claims 1-11 for the use in a method of inducing an immune response to at least one predetermined antigen in a subject, the use comprising administering to the subject a CAR-NK cell-derived effector cell population of any one of claims 1-11 in an amount sufficient to induce an anti-tumor response to a cancer in the subject, wherein the cancer expresses the predetermined antigen, wherein the immune response is preferably a cytotoxic anti-tumor response and/or, wherein the method preferably further comprises inducing tumor-cell apoptosis.

13. The CAR natural killer (NK) cell-derived effector cell population for the use of claim 12, wherein the cancer is selected from the group consisting of lung cancer, melanoma, breast cancer, prostate cancer, colon cancer, renal cell carcinoma, ovarian cancer, neuroblastoma, rhabdomyosarcoma, leukemia and lymphoma.

14. A method of preparing a CAR-NK cell-derived cell population comprising a population of NK cells expressing a chimeric antigen receptor (CAR), the CAR comprising an extracellular domain which specifically binds a predetermined antigen, comprising:
contacting *in vitro* one or more NK cells that have been modified to express the CAR with a stimulus that induces expansion of the NK cells to provide an expanded NK cell population; and
modifying the nucleic acid of the NK cells in the expanded NK cell population by reaction with a nucleic acid targeting compound that reacts directly with the nucleic acid,
wherein the nucleic acid targeting compound is a psoralen or a nucleic acid alkylator,
wherein the NK cells in the expanded NK cell population are preferably attenuated fo proliferation and/or,
wherein the method further comprises:
prior to or following the modifying step, activating *in vitro* the NK cells to produce a CAR-NK cell-derived effector cell population, wherein the NK cells in the effector NK cell population are preferably attenuated for proliferation.

15. The method of claim 14 wherein the method comprises contacting the one or more NK cells with the predetermined antigen under conditions in which the NK cells are both induced to expand and are activated by the same stimulus, and the NK cells in the expanded NK cell population are attenuated for proliferation following expansion and activation or, wherein the stimulus that induces expansion of the NK cells is a non-specific expansion stimulus, and wherein the expanded NK cell population is subsequently activated by contacting the NK cells in the expanded NK cell population with the predetermined antigen under conditions in which the NK cells are activated, wherein the NK cells in the expanded NK cell population are preferably attenuated for proliferation following expansion and activation and/or, wherein the stimulus comprises one or more of IL-2, IL-12, IL-15 and IL-18.

16. The method of any one of claims 14-15 wherein the nucleic acid targeting compound is a nucleic acid alkylator, wherein the nucleic acid alkylator is preferably a FRALE such as β-alanine, N-(acridin-9-yl), 2-[bis(2-chloroethyl)amino] ethyl ester.

17. The method of any one of claims 14-15, wherein the nucleic acid targeting compound is activated by illumination, wherein the nucleic acid targeting compound is preferably a psoralen compound activated by UVA illumination, wherein the psoralen is preferably 4'-(4-amino-2-oxa)butyl-4,5',8-trimethylpsoralen, 4'-aminomethyl-4,5',8-trimethylpsoralen (AMT), 5-methoxy psoralen, trioxalen, 4,5',8-trimethylpsoralen, or 8-methoxy psoralen.

18. The method of claim 17 wherein the NK cells comprise psoralen-induced interstrand crosslinks introduced between the strands of the genomic DNA, wherein the crosslinks preferably inhibit replication of the NK cells.

19. The method of any one of claims 14-18 wherein at least a portion of the NK cells produce one or more cytokines,
wherein preferably at least a portion of the NK cells produce one or more cytokines selected from the group consisting of IFN-γ, GM-CSF, TNF and IL-10.

20. The method of any one of claims 14-19, wherein at least a portion of the NK cells express one or more surface markers selected from the group consisting of CD56, CD16, CD27 and NKp46.

21. The method of any one of claims 14-20 wherein greater than 90% of the NK cells in the population are non-proliferating or, wherein greater than 90% of the activated NK cells in the population are non-proliferating.

22. The method of any one of claims 14-21, wherein the predetermined antigen is a cancer antigen, wherein the cancer is preferably selected from the group consisting of lung cancer, melanoma, breast cancer, prostate cancer, colon cancer, renal cell carcinoma, ovarian cancer, neuroblastoma, rhabdomyosarcoma, leukemia and lymphoma.

## Patentansprüche

1. Von CAR-Natürlichen-Killer (NK)-Zellen abgeleitete Effektor-Zellenpopulation, umfassend:
eine Population von NK-Zellen, die einen chimären Antigenrezeptor (CAR) exprimieren, wobei der CAR eine extrazelluläre Domäne umfasst, welche ein vorbestimmtes Antigen, eine Transmembrandomäne und eine zytoplasmatische kostimulatorische Signalisierungsdomäne spezifisch bindet, wobei die Nukleinsäure der NK-Zellen durch Reaktion mit einer auf die Nukleinsäure gerichteten Verbindung modifiziert worden ist, wobei die auf die Nukleinsäure gerichtete Verbindung ein Psoralen oder ein Nukleinsäurealkylator ist, wobei die NK-Zellen in der Population in einer therapeutisch wirksamen Menge für die Behandlung einer malignen Erkrankung vorliegen, die das vorbestimmte Antigen exprimiert.

2. Von CAR-NK-Zellen abgeleitete Effektor-Zellenpopulation nach Anspruch 1, wobei die Population von NK-Zellen, die einen chimären Antigenrezeptor exprimieren, eine Population von aktivierten NK-Zellen umfasst, die einen chimären Antigenrezeptor exprimieren.

3. Von CAR-NK-Zellen abgeleitete Effektor-Zellenpopulation nach Anspruch 1 oder 2, wobei die Reaktion der Nukleinsäure der NK-Zellen mit der auf die Nukleinsäure gerichteten Verbindung zu Quervernetzungen zwischen den Strängen und/oder Addukten in der Nukleinsäure führt, und/oder wobei die Nukleinsäure der NK-Zellen durch Reaktion mit der auf die Nukleinsäure gerichteten Verbindung modifiziert worden ist, sodass die NK-Zellen für die Proliferation abgeschwächt sind.

4. Von CAR-NK-Zellen abgeleitete Effektor-Zellenpopulation nach einem der Ansprüche 1 bis 3, wobei der Nukleinsäurealkylator ein FRALE (frangibler Anker-Linker-Effektor) wie z. B. β-Alanin, N-(Acridin-9-yl), 2-[bis(2-Chlorethyl)amino]ethylester ist.

5. Von CAR-NK-Zellen abgeleitete Effektor-Zellenpopulation nach einem der Ansprüche 1 bis 3, wobei die auf die Nukleinsäure gerichtete Verbindung durch Bestrahlung aktiviert wird, wobei die auf die Nukleinsäure gerichtete Verbindung vorzugsweise eine durch UVA-Bestrahlung aktivierte Psoralen-Verbindung ist, wobei das Psoralen vorzugsweise 4'-(4-Amino-2-oxa)butyl-4,5',8-trimethylpsoralen, 4'-Aminomethyl-4,5',8-trimethylpsoralen (AMT), 5-Methoxypsoralen, Trioxalen, 4,5',8-Trimethylpsoralen oder 8-Methoxypsoralen ist.

6. Von CAR-NK-Zellen abgeleitete Effektor-Zellenpopulation nach Anspruch 5, wobei die NK-Zellen durch Psoralen induzierte Quervernetzungen zwischen den Strängen umfassen, zwischen die Stränge der genomischen DNA eingebracht sind, wobei die Quervernetzungen zwischen den Strängen vorzugsweise die Replikation der NK-Zellen hemmen.

7. Von CAR-NK-Zellen abgeleitete Effektor-Zellenpopulation nach einem der Ansprüche 1 bis 6, wobei mindestens ein Teil der NK-Zellen ein oder mehrere Zytokine erzeugt, wobei mindestens ein Teil der NK-Zellen ein oder mehrere Zytokine erzeugt, die vorzugsweise aus der Gruppe bestehend aus IFN-γ, GM-CSF, TNF und IL-10 ausgewählt sind.

8. Von CAR-NK-Zellen abgeleitete Effektor-Zellenpopulation nach einem der Ansprüche 1 bis 7, wobei mindestens ein Teil der NK-Zellen ein oder mehrere Oberflächenmarker exprimiert, die aus der Gruppe bestehend aus CD56, CD16, CD27 und NKp46 ausgewählt sind.

9. Von CAR-NK-Zellen abgeleitete Effektor-Zellenpopulation nach einem der Ansprüche 1 bis 8, wobei mehr als 90 % der NK-Zellen in der Population nicht-proliferierend sind.

10. Von CAR-NK-Zellen abgeleitete Effektor-Zellenpopulation nach einem der Ansprüche 2 bis 8, wobei mehr als 90 % der aktivierten NK-Zellen in der Population nicht-proliferierend sind.

11. Von CAR-NK-Zellen abgeleitete Effektor-Zellenpopulation nach einem der Ansprüche 1 bis 10, wobei das vorbestimmte Antigen ein Krebsantigen ist, wobei der Krebs vorzugsweise aus der Gruppe bestehend aus Lungenkrebs, Melanom, Brustkrebs, Prostatakrebs, Darmkrebs, Nierenzellkarzinom, Ovarialkrebs, Neuroblastom, Rhabdomyosarkom, Leukämie und Lymphom ausgewählt ist.

12. Von CAR-Natürlichen-Killer (NK)-Zellen abgeleitete Effektor-Zellenpopulation nach einem der Ansprüche 1 bis 11 für die Verwendung bei einem Verfahren zum Bewirken einer Immunreaktion auf mindestens ein vorbestimmtes Antigen bei einem Individuum, wobei die Verwendung das Verabreichen einer von CAR-Natürlichen-Killer (NK)-Zellen abgeleiteten Effektor-Zellenpopulation nach einem der Ansprüche 1 bis 11 in einer für das Bewirken einer Anti-Tumor-Reaktion gegen einen Krebs in dem Individuum ausreichenden Menge an das Individuum umfasst, wobei der Krebs das vorbestimmte Antigen exprimiert, wobei die Immunreaktion vorzugsweise eine zytotoxische Anti-Tumor-Reaktion ist, und/oder wobei das Verfahren vorzugsweise weiterhin das Bewirken einer Tumorzellen-Apoptose umfasst.

13. Von CAR-Natürlichen-Killer (NK)-Zellen abgeleitete Effektor-Zellenpopulation für die Verwendung nach Anspruch 12, wobei der Krebs aus der Gruppe bestehend aus Lungenkrebs, Melanom, Brustkrebs, Prostatakrebs, Darmkrebs, Nierenzellkarzinom, Ovarialkrebs, Neuroblastom, Rhabdomyosarkom, Leukämie und Lymphom ausgewählt ist.

14. Verfahren zum Herstellen einer von CAR-NK-Zellen abgeleiteten Effektor-Zellenpopulation, umfassend eine Population von NK-Zellen, die einen chimären Antigenrezeptor (CAR) exprimieren, wobei der CAR eine extrazelluläre Domäne umfasst, welche ein vorbestimmtes Antigen spezifisch bindet, umfassend:
*In* vitro-Inkontaktbringen einer oder mehrerer NK-Zellen, die modifiziert worden sind, um den CAR zu exprimieren, mit einem Stimulus, der eine Expansion der NK-Zellen bewirkt, um eine expandierte NK-Zellenpopulation zu erhalten; und
Modifizieren der Nukleinsäure der NK-Zellen in der expandierten NK-Zellenpopulation durch Reaktion mit einer auf die Nukleinsäure gerichteten Verbindung, die direkt mit der Nukleinsäure reagiert,
wobei die auf die Nukleinsäure gerichtete Verbindung ein Psoralen oder ein Nukleinsäurealkylator ist,
wobei die NK-Zellen in der expandierten NK-Zellenpopulation vorzugsweise für die Proliferation abgeschwächt sind, und/oder
wobei das Verfahren weiterhin umfasst:
vor oder nach dem Modifizierungsschritt: *In vitro-*Aktivieren der NK-Zellen, um eine von CAR-NK-Zellen abgeleitete Effektor-Zellenpopulation zu erzeugen, wobei die NK-Zellen in der Effektor-NK-Zellenpopulation vorzugsweise für die Proliferation abgeschwächt werden.

15. Verfahren nach Anspruch 14, wobei das Verfahren das Inkontaktbringen der einen oder mehreren NK-Zellen mit dem vorbestimmten Antigen unter Bedingungen umfasst, bei denen bewirkt wird, dass die NK-Zellen sowohl expandiert als auch durch den gleichen Stimulus aktiviert werden, und die NK-Zellen in der expandierten NK-Zellenpopulation für die Proliferation nach der Expansion und Aktivierung abgeschwächt werden, oder wobei der Stimulus, der die Expansion der NK-Zellen bewirkt, ein nicht- spezifischer Expansionsstimulus ist, und wobei die expandierte NK-Zellenpopulation daraufhin durch Inkontaktbringen der NK-Zellen in der expandierten NK-Zellenpopulation mit dem vorbestimmten Antigen unter Bedingungen aktiviert wird, bei denen die NK-Zellen aktiviert werden, wobei die NK-Zellen in der expandierten NK-Zellenpopulation vorzugsweise für die Proliferation nach Expansion und Aktivierung abgeschwächt werden, und/oder wobei der Stimulus eines oder mehrere von IL-2, IL-12, IL-15 und IL-18 umfasst.

16. Verfahren nach einem der Ansprüche 14 bis 15, wobei die auf die Nukleinsäure gerichtete Verbindung ein Nukleinsäurealkylator ist, wobei der Nukleinsäurealkylator vorzugsweise ein FRALE wie z. B. β-Alanin, N-(Acridin-9-yl), 2-[bis(2-Chlorethyl)amino]ethylester ist.

17. Verfahren nach einem der Ansprüche 14 bis 15, wobei die auf die Nukleinsäure gerichtete Verbindung durch Bestrahlung aktiviert wird, wobei die auf die Nukleinsäure gerichtete Verbindung vorzugsweise eine durch UVA-Bestrahlung aktivierte Psoralen-Verbindung ist, wobei das Psoralen vorzugsweise 4'-(4-Amino-2-oxa)butyl-4,5',8-trimethylpsoralen, 4'-Aminomethyl-4,5',8-trimethylpsoralen (AMT), 5-Methoxypsoralen, Trioxalen, 4,5',8-Trimethylpsoralen oder 8-Methoxypsoralen ist.

18. Verfahren nach Anspruch 17, wobei die NK-Zellen durch Psoralen induzierte Quervernetzungen zwischen den Strängen umfassen, die zwischen die Stränge der genomischen DNA eingebracht werden, wobei die Quervernetzungen vorzugsweise die Replikation der NK-Zellen hemmen.

19. Verfahren nach einem der Ansprüche 14 bis 18, wobei mindestens ein Teil der NK-Zellen ein oder mehrere Zytokine erzeugt, wobei vorzugsweise mindestens ein Teil der NK-Zellen ein oder mehrere Zytokine erzeugt, die aus der Gruppe bestehend aus IFN-γ, GM-CSF, TNF und IL-10 ausgewählt werden.

20. Verfahren nach einem der Ansprüche 14 bis 19, wobei mindestens ein Teil der NK-Zellen ein oder mehrere Oberflächenmarker exprimiert, die aus der Gruppe bestehend aus CD56, CD16, CD27 und NKp46 ausgewählt werden.

21. Verfahren nach einem der Ansprüche 14 bis 20, wobei mehr als 90 % der NK-Zellen in der Population nicht-proliferierend sind, oder wobei mehr als 90 % der aktivierten NK-Zellen in der Population nicht-proliferierend sind.

22. Verfahren nach einem der Ansprüche 14 bis 21, wobei das vorbestimmte Antigen ein Krebsantigen ist, wobei der Krebs vorzugsweise aus der Gruppe bestehend aus Lungenkrebs, Melanom, Brustkrebs, Prostatakrebs, Darmkrebs, Nierenzellkarzinom, Ovarialkrebs, Neuroblastom, Rhabdomyosarkom, Leukämie und Lymphom ausgewählt wird.

## Revendications

1. Population de cellules effectrices dérivées de cellules CAR-NK (natural killer) comprenant :
une population de cellules NK exprimant un récepteur antigénique chimérique (CAR), le CAR comprenant un domaine extracellulaire qui se lie spécifiquement à un antigène prédéterminé, un domaine transmembranaire, et un domaine de signalisation de co-stimulation cytoplasmique, dans laquelle l'acide nucléique des cellules NK a été modifié par réaction avec un composé ciblant l'acide nucléique, dans laquelle le composé ciblant l'acide nucléique est un psoralène ou un alkylateur d'acide nucléique, dans laquelle les cellules NK sont présentes dans la population en une quantité thérapeutiquement efficace pour le traitement d'une malignité qui exprime l'antigène prédéterminé.

2. Population de cellules effectrices dérivées de cellules CAR-NK selon la revendication 1, dans laquelle la population de cellules NK exprimant un récepteur antigénique chimérique comprend une population de cellules NK activées exprimant un récepteur antigénique chimérique.

3. Population de cellules effectrices dérivées de cellules CAR-NK selon la revendication 1 ou 2 dans laquelle la réaction de l'acide nucléique des cellules NK avec le composé ciblant l'acide nucléique résulte en des réticulations inter-brins et/ou des produits d'addition dans l'acide nucléique et/ou, dans laquelle l'acide nucléique des cellules NK a été modifié par réaction avec le composé ciblant l'acide nucléique de telle manière que les cellules NK sont atténuées pour la prolifération.

4. Population de cellules effectrices dérivées de cellules CAR-NK selon l'une quelconque des revendications 1 à 3 dans laquelle l'alkylateur d'acide nucléique est un FRALE (frangible anchor linker effector) tel qu'une β-alanine, un N-(acridin-9-yl), un ester 2-[bis(2-chloroéthyl) amino]éthylique.

5. Population de cellules effectrices dérivées de cellules CAR-NK selon l'une quelconque des revendications 1 à 3 dans laquelle le composé ciblant l'acide nucléique est activé par éclairage, dans laquelle le composé ciblant l'acide nucléique est de préférence un composé psoralène activé par éclairage UVA, dans laquelle le psoralène est de préférence un 4'-(4-amino-2-oxa)butyl-4,5',8-triméthylpsoralène, un 4'-aminométhyl-4,5',8-triméthylpsoralène (AMT), un 5-méthoxy psoralène, un trioxalen-4,5'8-triméthylpsoralène, ou un 8-méthoxy psoralène.

6. Population de cellules effectrices dérivées de cellules CAR-NK selon la revendication 5 dans laquelle les cellules NK comprennent des réticulations inter-brins induites par un psoralène introduites entre les brins de l'ADN génomique, dans laquelle les réticulations inter-brins inhibent de préférence la réplication des cellules NK.

7. Population de cellules effectrices dérivées de cellules CAR-NK selon l'une quelconque des revendications 1 à 6, dans laquelle au moins une partie des cellules NK produisent une ou plusieurs cytokines, dans laquelle au moins une partie des cellules NK produisent une ou plusieurs cytokines de préférence sélectionnées dans le groupe constitué de l'IFN-y, du GM-CSF, du TNF et de l'IL-10.

8. Population de cellules effectrices dérivées de cellules CAR-NK selon l'une quelconque des revendications 1 à 7, dans laquelle au moins une partie de cellules NK exprime un ou plusieurs marqueurs de surface sélectionnés dans le groupe constitué de CD56, CD16, CD27 et NKp46.

9. Population de cellules effectrices dérivées de cellules CAR-NK selon l'une quelconque des revendications 1 à 8 dans laquelle plus de 90 % des cellules NK dans la population sont non proliférantes.

10. Population de cellules effectrices dérivées de cellules CAR-NK selon l'une quelconque des revendications 2 à 8 dans laquelle plus de 90 % des cellules NK activées dans la population sont non proliférantes.

11. Population de cellules effectrices dérivées de cellules CAR-NK selon l'une quelconque des revendications 1 à 10, dans laquelle l'antigène prédéterminé est un antigène du cancer, dans laquelle le cancer est de préférence sélectionné dans le groupe constitué d'un cancer du poumon, d'un mélanome, d'un cancer du sein, d'un cancer de la prostate, d'un cancer du côlon, d'un carcinome des cellules rénales, d'un cancer de l'ovaire, d'un neuroblastome, d'un rhabdomyosarcome, d'une leucémie et d'un lymphome.

12. Population de cellules effectrices dérivées de cellules CAR-NK (natural killer) selon l'une quelconque ces revendications 1 à 11 pour son utilisation dans un procédé d'induction d'une réponse immunitaire à au moins un antigène prédéterminé chez un sujet, l'utilisation comprenant l'administration au sujet d'une population de cellules effectrices dérivées de cellules CAR-NK selon l'une quelconque des revendications 1 à 11 en une quantité suffisante pour induire une réponse anti-tumorale à un cancer chez le sujet, dans laquelle le cancer exprime l'antigène prédéterminé, dans laquelle la réponse immunitaire est de préférence une réponse anti-tumorale cytotoxique et/ou, dans laquelle le procédé comprend de préférence en outre l'induction de l'apoptose des cellules tumorales.

13. Population de cellules effectrices dérivées de cellules CAR-NK (natural killer) pour son utilisation selon la revendication 12, dans laquelle le cancer est sélectionné dans le groupe constitué d'un cancer du poumon, d'un mélanome, d'un cancer du sein, d'un cancer de la prostate, d'un cancer du côlon, d'un carcinome des cellules rénales, d'un cancer de l'ovaire, d'un neuroblastome, d'un rhabdomyosarcome, d'une leucémie et d'un lymphome.

14. Procédé de préparation d'une population de cellules dérivées de cellules CAR-NK comprenant une population de cellules NK exprimant un récepteur antigénique chimérique (CAR), le CAR comprenant un domaine extracellulaire qui se lie spécifiquement à un antigène prédéterminé, comprenant :
la mise en contact *in vitro* d'une ou plusieurs cellules NK qui ont été modifiées pour exprimer le CAR avec un stimulus qui induit l'expansion des cellules NK pour fournir une population de cellules NK expansées ; et
la modification de l'acide nucléique des cellules NK dans la population de cellules NK expansées par réaction avec un composé ciblant l'acide nucléique qui réagit directement avec l'acide nucléique,
dans laquelle le composé ciblant l'acide nucléique est un psoralène ou un alkylateur d'acide nucléique,
dans laquelle les cellules NK dans la population de cellules NK expansées sont de préférence atténuées pour la prolifération et/ou,
dans laquelle le procédé comprend en outre :
avant ou après l'étape de modification, l'activation *in vitro* des cellules NK pour produire une population de cellules effectrices dérivées de cellules CAR-NK, dans lequel les cellules NK dans la population de cellules NK effectrices sont de préférence atténuées pour la prolifération.

15. Procédé selon la revendication 14, dans lequel le procédé comprend la mise en contact des une ou plusieurs cellules NK avec l'antigène prédéterminé dans des conditions dans lesquelles les cellules NK subissent à la fois une induction de leur expansion et une activation par le même stimulus, et les cellules NK dans la population de cellules NK expansées sont atténuées pour la prolifération après l'expansion et l'activation ou, dans lequel le stimulus qui induit l'expansion des cellules NK est un stimulus d'expansion non spécifique, et dans lequel la population de cellules NK expansées est ensuite activée par mise en contact des cellules NK dans la population de cellules NK expansées avec l'antigène prédéterminé dans des conditions dans lesquelles les cellules NK sont activées, dans lequel les cellules NK dans la population de cellules NK expansées sont de préférence atténuées pour la prolifération après l'expansion et l'activation et/ou, dans lequel le stimulus comprend un ou plusieurs de l'IL-2, de l'IL-12, de l'IL-15 et de l'IL-18.

16. Procédé selon l'une quelconque des revendications 14 et 15 dans lequel le composé ciblant l'acide nucléique est un alkylateur d'acide nucléique, dans lequel l'alkylateur d'acide nucléique est de préférence un FRALE tel qu'une β-alanine, un N-(acridin-9-yl), un ester 2-[bis(2-chloroéthyl)amino]éthylique.

17. Procédé selon l'une quelconque des revendications 14 et 15, dans lequel le composé ciblant l'acide nucléique est activé par éclairage, dans lequel le composé ciblant l'acide nucléique est de préférence un composé psoralène activé par éclairage UVA, dans lequel le psoralène est de préférence un 4'-(4-amino-2-oxa)butyl-4,5',8-triméthylpsoralène, un 4'-aminométhyl-4,5',8-triméthylpsoralène (AMT), un 5-méthoxy psoralène, un trioxalen-4,5',8-triméthylpsoralène, ou un 8-méthoxy psoralène.

18. Procédé selon la revendication 17 dans lequel les cellules NK comprennent des réticulations inter-brins induites par le psoralène introduites entre les brins de l'ADN génomique, dans lequel les réticulations inhibent de préférence la réplication des cellules NK.

19. Procédé selon l'une quelconque des revendications 14 à 18 dans lequel au moins une partie des cellules NK produisent une ou plusieurs cytokines,
dans lequel de préférence au moins une partie des cellules NK produisent une ou plusieurs cytokines sélectionnées dans le groupe constitué de l'IFN-y, du GM-CSF, du TNF et de l'IL-10.

20. Procédé selon l'une quelconque des revendications 14 à 19, dans lequel au moins une partie de cellules NK exprime un ou plusieurs marqueurs de surface sélectionnés dans le groupe constitué de CD56, CD16, CD27 et NKp46.

21. Procédé selon l'une quelconque des revendications 14 à 20 dans lequel plus de 90 % des cellules NK dans la population sont non proliférantes ou, dans lequel plus de 90 % des cellules NK activées dans la population sont non proliférantes.

22. Procédé selon l'une quelconque des revendications 14 à 21, dans lequel l'antigène prédéterminé est un antigène du cancer, dans lequel le cancer est de préférence sélectionné dans le groupe constitué d'un cancer du poumon, d'un mélanome, d'un cancer du sein, d'un cancer de la prostate, d'un cancer du côlon, d'un carcinome des cellules rénales, d'un cancer de l'ovaire, d'un neuroblastome, d'un rhabdomyosarcome, d'une leucémie et d'un lymphome.
